# EUROPEAN PATENT APPLICATION

(11) **EP 4 732 850 A1**
(43) Date of publication of application: **29.04.2026**
(21) Application number: 24315497.8
(22) Date of filing: 25.10.2024
(51) Int. Cl.: A61K 47/68, A61P 35/00

(54) **ANTIBODY-DRUG CONJUGATES AND THEIR USES**

(71) Applicant: Antelope Therapeutics SAS, 69300 Caluire-Et-Cuire (FR)
(72) Inventor: Viricel, Warren, 69160 Tassin (FR); Leroy, Edouard, 69700 Beauvallon (FR)
(74) Representative: Mewburn Ellis LLP

(57) **Abstract**

There are provided antibody-drug conjugates, wherein the antibody component specifically binds to CA242, and wherein the drug is a topoisomerase I inhibitor. Such antibody-drug conjugates are useful in particular in treating proliferative diseases including cancers, such as colorectal, pancreas, gastric and lung cancers.

## Description

### Technical field

The present disclosure relates to antibody-drug conjugates, and in particular where the antibody component specifically binds to the extracellular domain of a tumor-associated sialic acid-containing carbohydrate epitope named CA242 (also known as H-CanAg, L-CanAg or CanAg).

### Background

Antibody-drug conjugates (hereafter referred as "ADCs") are a class of therapeutics composed of an antibody and an attached drug (*e.g*. a cytotoxic drug or payload), covalently bonded by a chemical linker. ADCs combine the specificity of antibody tumor-targeting with ` the cell-killing efficiency of the payload (*e.g.* the cytotoxic activity of the attached chemotherapeutic drug). Efficient ADCs should exhibit high specificity and low systemic toxicity.

Payloads used in ADCs are highly potent with *in vitro* cell killing activities in the pM range. Common payloads are microtubule inhibitors (such as maytansine derivatives (DM1/DM4), auristatins (MMAE/MMAF), eribulin and DNA alkylators (such as calicheamicin, pyrrolobenzodiazepines, indolinobenzodiazepines, or duocarmycins). Those payloads are typically conjugated to antibodies with a drug-to-antibody ratio (DAR) of 2 to 4.

More recently, a new class of moderately potent payload with *in vitro* cell killing activities in the nM range, namely topoisomerase I inhibitors, has emerged. Those payloads are typically conjugated to the antibody with an increased DAR value of 8 to circumvent their lower intrinsic potency, compared to highly potent payload discussed above, and deliver a final ADC that is potent enough to show acceptable efficacy properties. Examples of such recent DAR 8 topoisomerase I inhibitor-based ADCs are trastuzumab deruxtecan (Enhertu^{®}), sacituzumab govitecan (Trodelvy^{®}), rinatabart sesutecan (PRO1184), M9140 investigational ADC from Merck KGaA, AZD8205 investigational ADC from AstraZeneca, patritumab deruxtecan and LY4101174 investigational ADC from Eli Lilly.

Because of the rapid binding of antibodies relative to their transport through tissue, often mediated by diffusion due to elevated interstitial pressure, ADCs must saturate the perivascular cells before penetrating deeper into the solid tumor tissue to exert acceptable prolonged efficacy (Rubahamya 2024). As this occurs, the cancer cells internalize and/or degrade the ADC, consuming the ADC before it can reach deeper in the tumor tissue.

Therefore, the concentration of ADC needed to saturate these perivascular cells (often referred to as the "binding site barrier") is the major factor in determining the maximum tissue penetration distance and overall efficacy. Decreasing the DAR (2-4 instead of 8) of the ADC while still using a moderately potent topoisomerase I inhibitor payload has the potential to increase 2 to 4 times the clinical dose of conjugate administered to the patient (while keeping a similar payload dose to maintain potency), which could result in a drastically improved clinical response rate.

CA242 (also known as H-CanAg or L-CanAg, as identified in Baeckström 1991 and US5552293A) is a carbohydrate epitope that is aberrantly overexpressed to various degrees by some human solid tumors (highly over-expressed in colorectal, pancreas, gastric and lung; expressed in a substantial proportion of non-small cell lung cancers, breast and bladder cancers) while being virtually absent from human healthy tissues. CA242 appears to be a suitable target for antibody-mediated delivery of anticancer agents.

In 2004 and 2007 respectively were reported phase I clinical trials of cantuzumab mertansine (huC242-DM1, see Helft 2004) and cantuzumab ravtansine (huC242-DM4, see Mita 2007), two ADCs targeting CA242. Those two trials were discontinued due to ADC platform related toxicities (liver toxicities for DM1, ocular toxicities for DM4), but no CA242-related ("on-target") toxicities were specifically identified, thereby confirming the cleanliness of the targeted approach. Limited signs of efficacy were detected in colorectal cancer patients, which could certainly be explained by the fact that colorectal cancers are known to be insensitive to mitotic inhibitors such as maytansines (O'Connell 1978).

New CA242-targeting ADCs based on pyrrolobenzodiazepines (PBD) and highly-potent DNA alkylator payloads were reported in 2022 (Mysliwy 2022 and WO2023/166322). Although efficacious in preclinical cancer models, the reported 1mg/kg highest non-severely toxic dose (HNSTD) in cynomolgus monkeys of those highly potent ADCs appears to be low when compared with 30-60mg/kg typical topoisomerase I inhibitor-based ADCs HNSTD's in cynomolgus monkeys that are commonly reported in the literature. As a result, the possibility of delivering high enough doses in the clinic to enhance solid tumor tissue penetration and promote sufficient efficacy seems limited with this approach.

There are currently no approved therapies that target CA242. In the past, two clinical trials of ADCs targeting CA242 have been reported: Phase I of cantuzumab mertansine and Phase I/II of cantuzumab ravtansine. Those trials have been since discontinued. It has been found that pairing CA242-targeting antibodies with topoisomerase I inhibitor payloads results in ADCs that are (i) highly efficacious, (ii) well tolerated and (iii) differentiated regarding their payload mechanism of action.

There therefore remains a need to design and develop an ADC comprising an anti-CA242 antibody component which is efficient, has high specificity and low toxicity, and that could be delivered at high enough doses in the clinic to promote solid tumor penetration and enhance efficacy. Such an ADC would be based on a differentiated mechanism of action of the payload (topoisomerase I inhibition) and would be designed with a lower drug-to-antibody ratio value (DAR 2 to 4) compared to higher DAR values that are typically used in the field (DAR 8).

### Summary

Accordingly, in a first aspect there is provided an antibody-drug conjugate (ADC) of formula (I):

Ab-[L-D]*i* (I)

wherein:
Ab is an anti-CA242 antibody as defined below,
L is a linker moiety bound to said anti-CA242 antibody,
D is a topoisomerase I inhibitor drug moiety bound to L,
*i* is from 1 to 8.

In a second aspect there is provided an ADC of the first aspect for use in a method of treatment. The second aspect also provides a pharmaceutical composition comprising the ADC of the first aspect, in combination with one or more pharmaceutical acceptable excipients, diluents or carriers.

In a third aspect there is provided a method of treating cancer, in a subject suffering therefrom, the method comprising administering to the subject an ADC of the first aspect. The third aspect also provides an ADC of the first aspect for use in method of treating cancer. The third aspect also provides the use of an ADC of the first aspect in the manufacture of a medicament for treating cancer.

In some aspects, there is used composition comprising a mixture of ADCs according to the first aspect, wherein the average i in the mixture of ADCs is about 1 to about 8.

### Detailed Description

### Antibody

The term "CA242" or "CanAg" or "L-CanAg" or "H-CanAg" refers to the sialylated Lewis glycopepitope antigen (carbohydrate) that is targeted by the antibodies and antibody drug conjugates described herein. This glycopepitope is described in Baeckström 1991 and in US patent US5552293A. This carbohydrate epitope is aberrantly overexpressed to various degrees by some human solid tumors while being virtually absent from human healthy tissues.

The term "antibody" means an immunoglobulin molecule that recognizes and specifically binds to a target, such as a protein, polypeptide, peptide, carbohydrate, polynucleotide, lipid, or combinations of the foregoing through at least one antigen recognition site within the variable region of the immunoglobulin molecule. References to antibodies include immunoglobulins whether natural or partly or wholly synthetically produced.

As used herein, the term "antibody" encompasses intact polyclonal antibodies, intact monoclonal antibodies, antibody fragments (such as Fab, Fab', F(ab')2, and Fv fragments), single chain Fv (scFv) mutants or multispecific antibodies such as bispecific antibodies.

A naturally occurring "antibody" is a protein comprising at least two heavy (H) chains and two light (L) chains inter-connected by disulfide bonds. Each heavy chain is comprised of a heavy chain variable region (abbreviated herein as VH) and a heavy chain constant region. Each light chain is comprised of a light chain variable region (abbreviated herein as VL) and a light chain constant region.

The VH and VL regions can be further subdivided into regions of hypervariability, termed complementarity determining regions (CDR), interspersed with regions that are more conserved, termed framework regions (FR). Each VH and VL is composed of three CDRs and four FRs. The exact location and identity of the 6 CDR sequences within the VH and VL may be determined using different definition schemes, such as the IMGT, Kabat, Chothia, and contact schemes (Dondelinger 2018). The CDR sequences disclosed herein as SEQ ID Nos: 1 to 6 have been determined using the IMGT definition scheme.

The variable regions of the heavy and light chains contain a binding domain that interacts with an antigen. The constant regions of the antibodies may mediate the binding of the immunoglobulin to host tissues or factors, including various cells of the immune system (e.g. effector cells) and the first component (Clq) of the classical complement system.

The term "antigen-binding portion" of an antibody (or simply "antigen portion"), as used herein, refers to full length or one or more fragments of an antibody that retain the ability to specifically bind to an antigen (*e.g*. a portion of CA242).

As used herein, the term "affinity" refers to the strength of interaction between antibody and antigen at single antigenic sites. Within each antigenic site, the variable region of the antibody "arm" interacts through non-covalent forces with the antigen at numerous sites; the more interactions, the stronger the affinity.

The phrases "an antibody recognizing an antigen" and "an antibody specific for an antigen" are used interchangeably herein with the term "an antibody which binds specifically to an antigen". For example, specific binding to an antigen may be used to mean the antibody binds the antigen with a higher affinity than a non-specific partner such as Bovine Serum Albumin (BSA, Genbank accession no. CAA76847, version no. CAA76847.1 GI:3336842, record update date: Jan 7, 2011 02:30 PM). In some cases, the antibody binds the antigen with an association constant (Kₐ) at least 2, 3, 4, 5, 10, 20, 50, 100, 200, 500, 1000, 2000, 5000, 10⁴, 10⁵ or 10⁶-fold higher than the antibody's association constant for BSA, when measured at physiological conditions. The antibodies described can bind the antigen with a high affinity. For example, in some embodiments the antibody can bind the antigen with a K_{D} equal to or less than about 10⁻⁶ M, such as 1 × 10⁻⁶, 10⁻⁷, 10⁻⁸, 10⁻⁹, 10⁻¹⁰, 10⁻¹¹, 10⁻¹², 10-¹³ or 10⁻¹⁴.

The terms "monoclonal antibody" or "monoclonal antibody composition" as used herein refers to a preparation of antibody molecules of uniform properties. A monoclonal antibody composition displays a single binding specificity and affinity for one specific epitope.

The antibody can be of any isotype (e.g. IgG, IgE, IgM, IgD, and IgA), class (e.g. IgG1, IgG2, IgG3, IgG4, IgA1 and IgA2) or subclass, or allotype (e.g. human G1m1, G1m2, G1m3, non-G1m1 [that, is any allotype other than G1m1], G1m17, G2m23, G3m21, G3m28, G3m11, G3m5, G3m13, G3m14, G3m10, G3m15, G3m16, G3m6, G3m24, G3m26, G3m27, A2m1, A2m2, Km1, Km2 and Km3) of antibody molecule. In some embodiments, Ab is an antibody comprising a human IgG1 isotype constant region.

In some embodiments, Ab comprises a heavy chain constant region having a substitution of the leucine residue at position 234 in the EU index set forth in Kabat and/or a substitution of the leucine residue at position 235 in the EU index set forth in Kabat. Typically the leucine residue is substituted for an alanine residue. In some embodiments both of the leucine residues at position 234 and 235 in the EU index set forth in Kabat are substituted for alanine residues. Such embodiments are termed 'LALA' embodiments. An example of a LALA embodiment is SEQ ID NO: 42, where residues A117 and A118 (which correspond to Kabat positions 234 and 235) are the result of substitutions of the native leucine residues.

In some embodiments, Ab comprises a heavy chain constant region having a substitution of the alanine residue at position 118 in the EU index set forth in Kabat. Typically the alanine residue is substituted for a cysteine residue. Such embodiments are termed 'A118C' embodiments. An example of a A118C embodiment is SEQ ID NO: 43, where residue C1 (which corresponds to Kabat position 118) is the result of substitutions of the native alanine residue.

In some embodiments, Ab comprises a light chain constant region having a substitution of the valine residue at position 205 in the EU index set forth in Kabat. Typically the valine residue is substituted for a cysteine residue. Such embodiments are termed 'V205C' embodiments. An example of a V205C embodiment is SEQ ID NO: 44, where residue V98 (which corresponds to Kabat position 205) is the result of substitution of the native valine residue.

In some embodiments, Ab is an anti-CA242 antibody comprising either:
(a) a variable heavy chain polypeptide comprising CDR-H1 of SEQ ID NO:1, CDR-H2 of SEQ ID NO:2, CDR-H3 of SEQ ID NO:3 and a variable light chain polypeptide comprising CDR-L1 of SEQ ID NO:4, CDR-L2 of SEQ ID NO:5 and CDR-L3 of SEQ ID NO:6; or,
(b) a variable heavy chain polypeptide comprising CDR-H1, CDR-H2, and CDR-H3 of SEQ ID NO:7, and a variable light chain polypeptide comprising CDR-L1, CDR-L2, and CDR-L3 of SEQ ID NO:8; or,
(c) a variable heavy chain polypeptide selected from VH of SEQ ID NO:13, SEQ ID NO:14, SEQ ID NO:15, SEQ ID NO:16 or SEQ ID NO:17 and a variable light chain polypeptide selected from VL of SEQ ID NO:18, SEQ ID NO:19, SEQ ID NO:20, SEQ ID NO:21 or SEQ ID NO:22.

In some embodiments, Ab is an anti-CA242 antibody comprising:
(a) a constant heavy chain polypeptide selected from SEQ ID NO: 41 or SEQ ID NO: 42; and
(b) a constant light chain polypeptide selected from SEQ ID NO: 43 or SEQ ID NO: 44.

In some embodiments, Ab is an anti-CA242 antibody comprising
(a) a constant heavy chain polypeptide of SEQ ID NO: 42; and
(b) a constant light chain polypeptide of SEQ ID NO: 44.

Any humanized antibody or an antigen-binding fragment thereof that targets CA242 may be used. In order to maintain binding affinity, the humanization process may comprise identifying CDR regions and residues interacting with CDRs and apply humanization procedures that preserve interaction with the antigen. Antibodies for use herein are humanized antibodies in which CDR sequences are composed of CDR-H1 of SEQ ID NO:1, CDR-H2 of SEQ ID NO:2, CDR-H3 of SEQ ID NO:3, CDR-L1 of SEQ ID NO:4, CDR-L2 of SEQ ID NO:5 and CDR-L3 of SEQ ID NO:6.

Embodiments of humanized anti-CA242 antibodies may comprise one or more amino acid sequences from SEQ ID NO: 13-17 or SEQ ID NO:18-22.

In some embodiments, Ab is an anti-CA242 antibody comprising:
(a) a heavy chain polypeptide selected from SEQ ID NO: 23, SEQ ID NO: 24, SEQ ID NO: 25, SEQ ID NO: 26, SEQ ID NO: 27 or SEQ ID NO: 28; and
(b) a light chain polypeptide selected from SEQ ID NO: 31, SEQ ID NO: 32, SEQ ID NO: 33, SEQ ID NO: 34, or SEQ ID NO: 35.

In some embodiments, Ab is an anti-CA242 antibody comprising
(a) a constant heavy chain polypeptide of SEQ ID NO: 26; and
(b) a constant light chain polypeptide of SEQ ID NO: 35.

### Linker

As used herein, the term "linker" or L in the formula (I) of the ADC refers to the non-targeting chemical moiety portion of an antibody-drug conjugate disclosed herein that covalently bond together the antibody component (Ab) and the drug component (D).

L may comprise an optional spacer or "stretcher unit" linked to Ab, or which is part of the linker L.

L may comprise a cleavable moiety, which is linked to D, or which is part of the linker L.

In some embodiments, L is a linker moiety of formula -W-Y-, wherein W is a stretcher unit linked to Ab, and Y is a cleavable unit linked to D.

The term "spacer" or "stretcher" unit or W refers to a component that connects different parts of the compound together, for example, the stretcher can connect Ab to a linker L or be part of linker L.

The group W may comprise a conjugated moiety, that is a group resulting from conjugation of the linker to the antibody. In some embodiments, this is selected from:

| | | | |
|---|---|---|---|
| (W¹) | 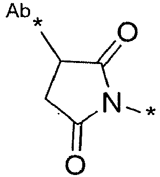 | (W⁹) | 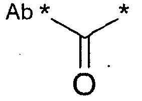 |
| (W²) | 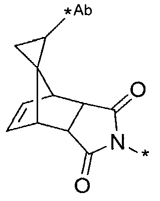 | (W¹⁰) | 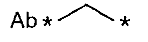 |
| (W³) | 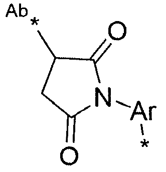 | (W¹¹) | 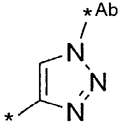 |
| (W⁴) | 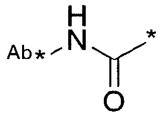 | (W¹²) | 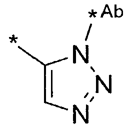 |
| (W⁵) | 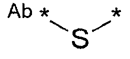 | (W¹³) | 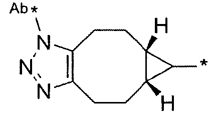 |
| (W⁶) | 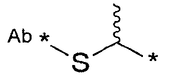 | (W¹⁴) | 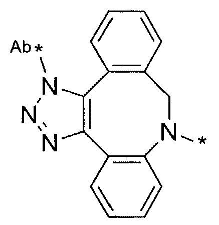 |
| (W7) | 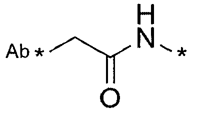 | (W¹⁵) | 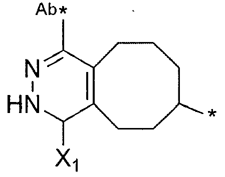 |
| (W⁸) | 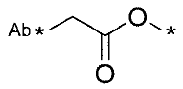 | | |

where Ab* indicates the point of attachment to the antibody, * indicates the point of attachment to the remainder of W, Ar represents a C₅₋₆ arylene group, e.g. phenylene, and X¹ represents C₁₋₄ alkyl.

In some embodiments, the conjugated moiety is selected from W¹ and W³.

In some embodiments, the conjugated moiety is W¹.

The remainder of W may comprise a variety of groups such as C₁-C₁₀ alkylene groups ([CH₂]ₙ, where n is 1 to 10), PEG groups ([C₂H₄O]ₙ, where n is 1 to 16), -C₂H₄-NHC(=O)-, polar acyl sulfamide groups (-S(=O)₂NH-C(=O)-), polar carbamoyl sulfamide groups (-NHS(=O)₂NH-C(=O)-).

In some embodiments, the remainder of W may be of the following formula: 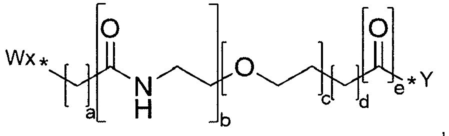 where a = 0 to 5, b = 0 or 1, c = 0 to 16, d = 0 to 5, e is 0 or 1, *Y indicates the point of attachment to Y, and Wx* indicates the point of attachment to the conjugated moiety.

In some embodiments, a is 0 to 2, such as 0, 1 or 2. In some of these embodiments a is 2.

In some embodiments, b is 0. In other embodiments, b is 1.

In some embodiments, c is 0 to 8. In some embodiments, c is 0, 1, 4 or 8. In some of these embodiments, c is 0. In other of these embodiments, c is 1. In other of these embodiments, c is 4. In other of these embodiments, c is 8.

In some embodiments, d is 0 to 2, such as 0, 1 or 2. In some of these embodiments, d is 0.

In some embodiments, e is 0. In other embodiments, e is 1.

In some embodiments:
(i) a is 2, b is 0, c is 4, d is 0 and e is 1; or
(ii) a is 2, b is 0, c is 1, d is 0 and e is 1; or
(iii) a is 2, b is 0, c is 0, d is 0 and e is 1; or
(iv) a is 2, b is 1, c is 8, d is 0 and e is 1.

In some embodiments, the stretcher unit (W) is selected from:

| | |
|---|---|
| 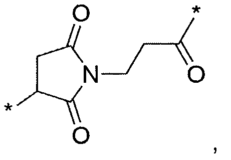 | |
| 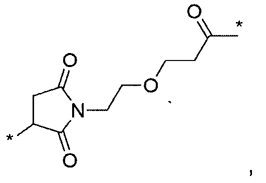 | |
| 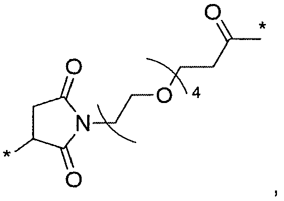 | and |
| 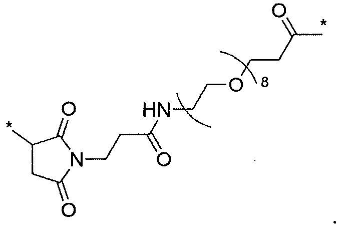 | |

As used herein, the term "cleavable unit" or Y refers to a chemical moiety that is cleaved upon enzymatic or chemical treatment to release drug unit D.

In some embodiments, the cleavable unit is a chemical cleavage unit.

In some embodiments, the cleavable unit is an acid-cleavable unit, *e.g*. comprising a hydrazone group (*e.g*. a 6-maleimidocaproylhydrazone linker or a (4-(4-acetylphenoxy)butanoic acid) hydrazone linker), a carbonate group or a silyl ether group. In some embodiments, the cleavable unit is an acid-cleavable unit is a Fe(II)-cleavable linker, *e.g.* comprising a 1,2,4-trioxolane group.

In some embodiments, the cleavable unit is an enzyme cleavage unit.

In some embodiments, the cleavable unit is a GSH-cleavable linker, *e.g*. comprising a disulfide group.

In some embodiments, the cleavable unit is a cathepsin-cleavable unit, *e.g.* comprising a dipeptide (*e.g*. valine-citrulline, phenylalanine-lysine or valine-alanine), a triglycyl peptide (CX), alanine-alanine-asparagine (Ala-Ala-Asn), a cBu-Cit group, or glycine-glycine-phenylalaline-glycine (GGFG).

In some embodiments, the cleavable unit is a galactosidase-cleavable unit, *e.g.* comprising a β-galactoside group.

In some embodiments, the cleavable unit is a phosphatase-cleavable unit, *e.g.* comprising a pyrophosphate group.

In some embodiments, the cleavable unit is a sulfatase-cleavable unit, *e.g.* comprising an arylsulfate group.

In some embodiments, the cleavable unit Y comprises a self-immolative group which links the cleavable moiety to the drug. In some of these embodiments, the self-immolative group is selected from para-aminobenzyl carbamate (PABC) and para-aminobenzyl (PAB).

In some embodiments, the cleavable unity comprises a glutamic acid derived group (-NHCH(CO₂H)CH₂C(=O)-) or a glutamic amide derived group, where the amide bears a methyl-PEG group with 1 to 6 ethyleneoxy groups. Such groups can be represented as: 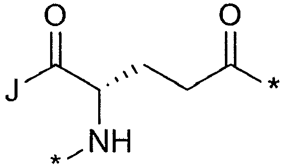 where J is OH or -NH-(C₂H₄O)_{f1}-Me, where f1 is from 1 to 6.

In some embodiments, L is of formula (III): 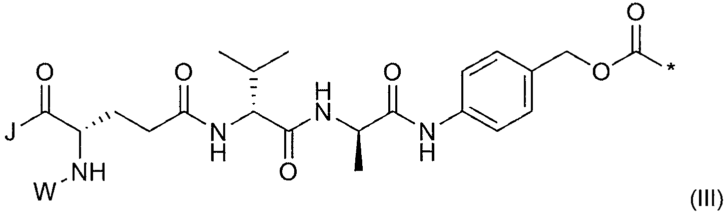 wherein J is OH or -NH-(C₂H₄O)_{f1}-Me, where f1 is from 1 to 6, * represents the attachment to the drug and W is as defined above.

In some of these embodiments f1 is 4 to 6. In particular, f1 can be 5.

In some embodiments, L is of formula (IV): 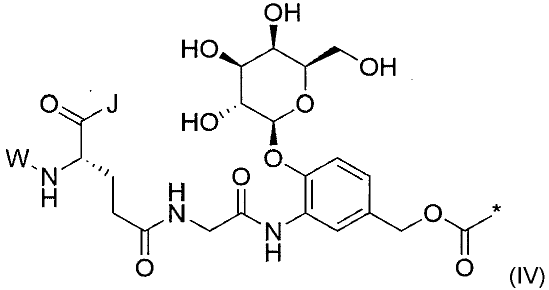 wherein J is OH or -NH-(C₂H₄O)_{f2}-Me, where f2 is from 1 to 6, * represents the attachment to the drug and W is as defined above.

In some of these embodiments f2 is 4 to 6. In particular, f2 can be 5.

In some embodiments, L is of formula (V): 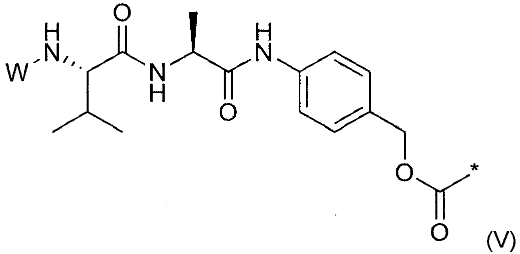 wherein * represents the attachment to the drug and W is as defined above.

### Drug

As used herein, the term "drug" or D in the formula (I) of the ADC refers to a topoisomerase I inhibitor.

As used herein, the term "topoisomerase I inhibitors" refers to a class of chemical compounds that block the action of Type I topoisomerases to exert cytotoxic activity. Known topoisomerase I inhibitors are camptothecin derivatives (such as 7-Ethyl-10-hydroxy-camptothecin known as SN-38, exatecan, exatecan mesylate (DX-8951f), *N*-glycyl-exatecan, DXd, irinotecan, etirinotecan, topotecan, gimatecan, belotecan, deruxtecan, belotecan, rubitecan, lurtotecan, diflomotecan, karenitecan, silatecan, namitecan, elomotecan, delimotecan, chimmitecan, indotecan (LMP-400), indimitecan (LMP-776), AZ14170132, Ed-04, ZD06519, YL0014, SHR9265, KL610023, FL118, DRF-1042, NSC606985, ZBH-1205, Genz-644282, SHR9265, A1.9, P1003, P1021, VIP126, ZBH-01 and LMP-744) and non-camptothecin derivatives (such as indenoisoquinoline, fluoroindenoisoquinoline, indolocarbazoles or phenanthridine derivatives).

In some embodiments, D is a camptothecin analogue. In some of these embodiments, D is derived from exatecan, i.e. is of formula (Ila) below:

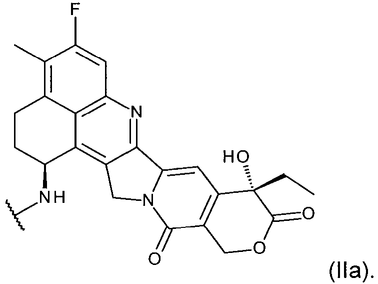

In some of these embodiments, D is derived from belotecan, i.e. is of formula (lib) below:

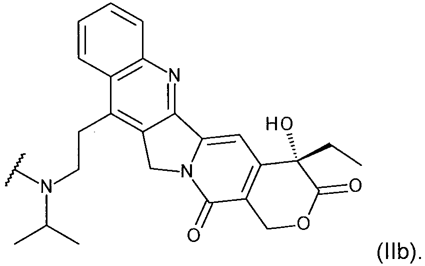

In some of these embodiments, D is derived from SN38, i.e. is of formula (IIc) below:

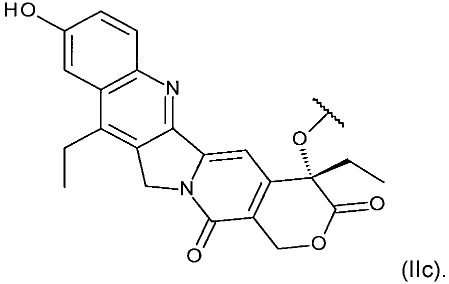

In some of these embodiments, D is derived from DXd, i.e. is of formula (IId) below:

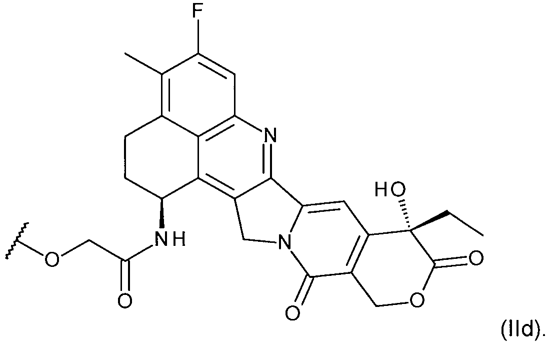

### Coupling

Coupling D to the antibody via the linker moiety (L) may be limited by the number of attachments sites on the antibody moiety. In some embodiments, L attaches to the antibody moiety through a chemically active group on one or more amino acid residues on the antibody moiety. For example, the linker may be attached to the antibody moiety via a free amino acid, imino, hydroxyl, thiol or carboxyl group (e.g. to the N- or C- terminus, to the epsilon amino group of one or more lysine residues or to the sulfhydryl group of one or more cysteine residues). The site to which L is attached can be natural residue in the amino acid sequence of the antibody moiety (e.g. native cysteines of the hinge region of antibody), or it can be introduced into the antibody moiety by DNA recombinant technology or any other technology (e.g. by introducing a cysteine or a non-natural amino acid residue into the amino acid sequence of the antibody).

In one embodiment, L is bonded to thiol reactive residues of the antibody.

In one embodiment, L is bonded to thiol reactive residues that have been site-specifically introduced into the antibody moiety by DNA recombinant technology.

### Drug-Antibody Ratio

For each individual ADC, *i* is the number of drug-linkers attached to the antibody and is an integer between 1 and 8.

In some embodiments, *i* is 2 to 4.

In some embodiments, *i* is 4.

In some embodiments, *i* is 2

Depending on how the ADC is synthesized, the resulting product is a mixture of ADC compounds with a distribution of drug moieties attached to the antibody, e.g. *i* is 1, 2, 3, etc.

Thus, there is provided a composition comprising a mixture of ADCs, wherein the average *i* in the mixture of ADCs is about 1 to about 8. In some embodiments, the average *i* is 2 to 4.

In some embodiments, the average *i* is 4.

In some embodiments, the average *i* is 2.

Liquid chromatography methods such as polymeric reverse phase (PLRP) and hydrophobic interaction (HIC) may separate compounds in the mixture by drug loading value (*i*).

Preparations of ADC with a single drug loading value (*i*) may be isolated, however, these single loading value ADCs may still be heterogeneous mixtures because the drug moieties may be attached, via the linker, at different sites on the antibody.

Thus, the antibody-drug conjugate compositions include mixtures of antibody-drug conjugate compounds where the antibody has one or more drug moieties and where the drug moieties may be attached to the antibody at various amino acid residues.

### Embodiments of Linking Group

In one embodiment, L is covalently bonded to one or more thiol residues of said antibody and said L is of formula (VI):

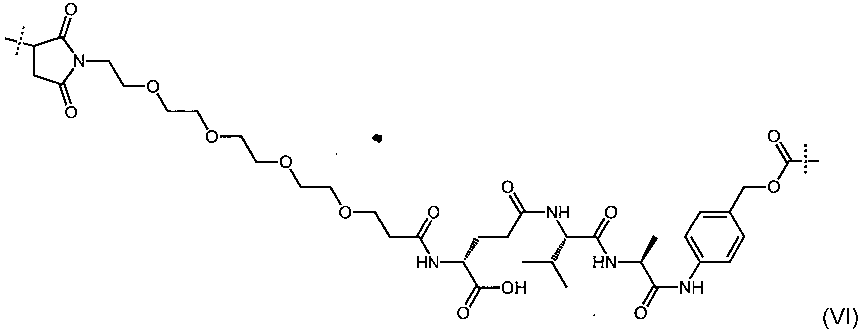

In one embodiment, L is covalently bonded to one or more thiol residues of said antibody and said L is of formula (VII): 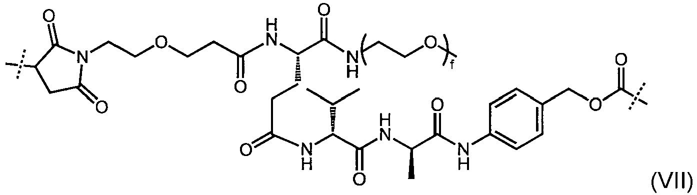 wherein f is an integer between 1 and 6.

In some of these embodiments, f is from 4 and 6, more preferably being 5.

In one embodiment, L is covalently bonded to one or more thiol residues of said antibody and said L is of formula (VIII):

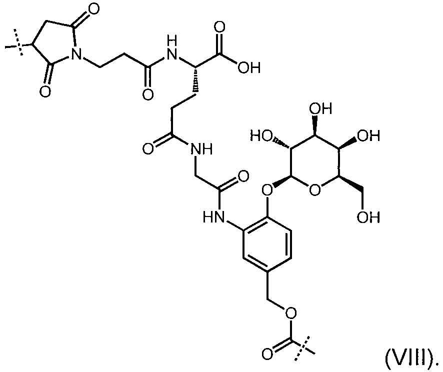

In one embodiment, L is covalently bonded to one or more thiol residues of said antibody and said L is of formula (IX):

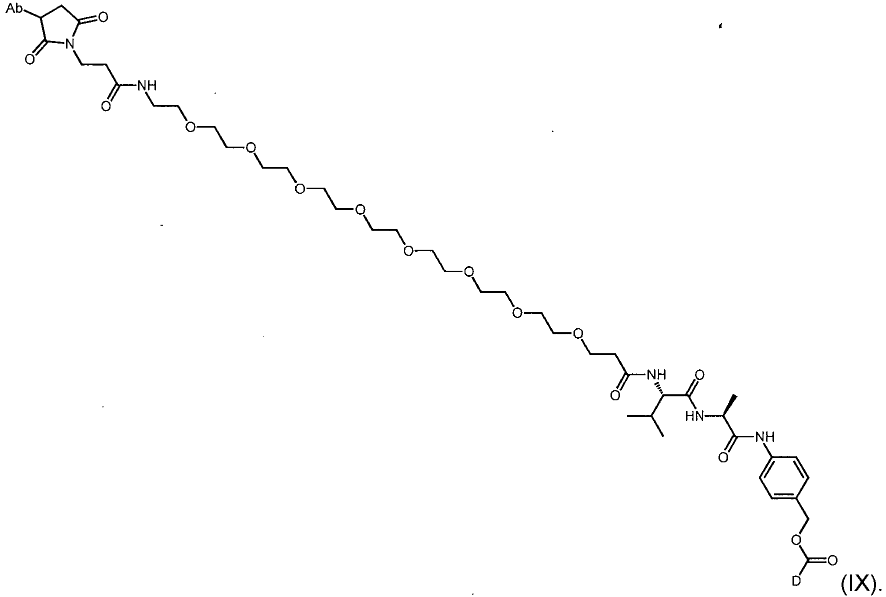

### Embodiments of the ADC

In one embodiment, the antibody drug conjugate is of formula (X): 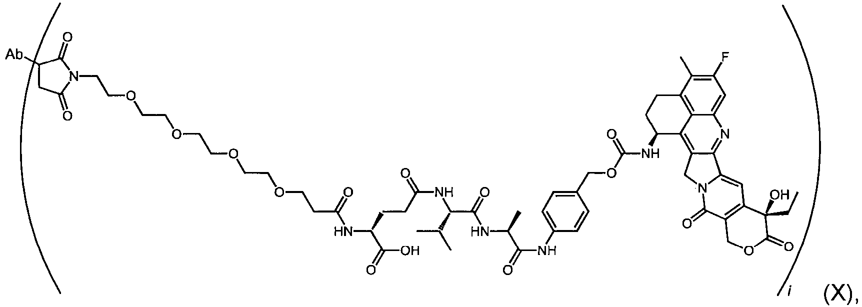 where *i* is from 1 to 8.

In some of these embodiments, Ab is an anti-CA242 antibody comprising a variable heavy chain polypeptide selected from VH of SEQ ID NO:13, SEQ ID NO:14, SEQ ID NO:15, SEQ ID NO:16 or SEQ ID NO:17 and comprising a variable light chain polypeptide selected from VL of SEQ ID NO:18, SEQ ID NO:19, SEQ ID NO:20, SEQ ID NO:21 or SEQ ID NO:22,

In some of these embodiments is from 2 to 4.

In one embodiment, the antibody drug conjugate is of formula (XI) 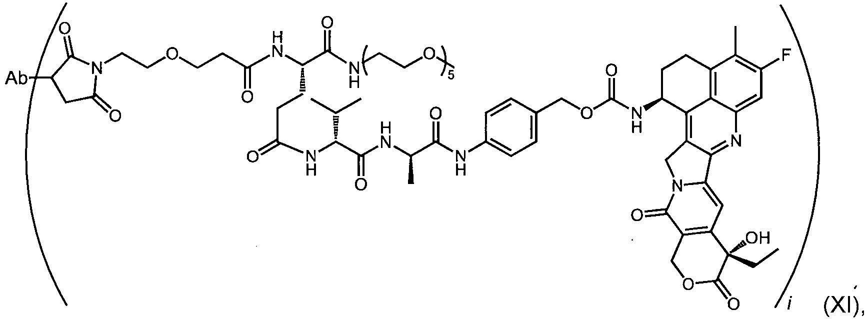 where i is from 1 to 8.

In some of these embodiments, Ab is an anti-CA242 antibody comprising a variable heavy chain polypeptide selected from VH of SEQ ID NO:13, SEQ ID NO:14, SEQ ID NO:15, SEQ ID NO:16 or SEQ ID NO:17 and comprising a variable light chain polypeptide selected from VL of SEQ ID NO:18, SEQ ID NO:19, SEQ ID NO:20, SEQ ID NO:21 or SEQ ID NO:22.

In some of these embodiments, i is from 2 to 4.

In one embodiment, the antibody drug conjugate corresponds to the following formula (XII) 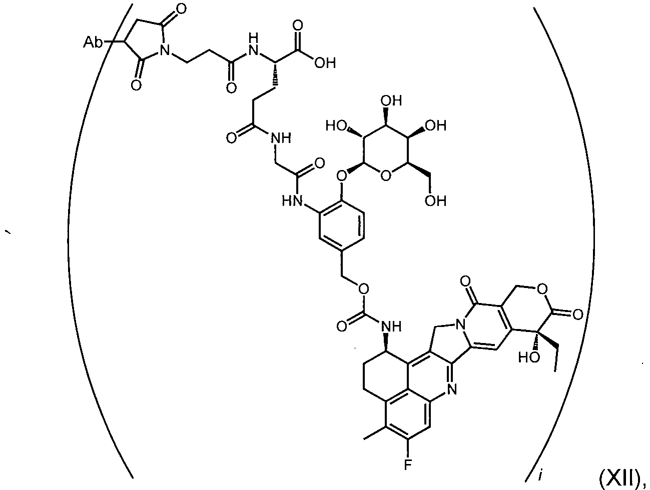 where *i* is from 1 to 8.

In some of these embodiments, Ab is an anti-CA242 antibody comprising a variable heavy chain polypeptide selected from VH of SEQ ID NO:13, SEQ ID NO:14, SEQ ID NO:15, SEQ ID NO:16 or SEQ ID NO:17 and comprising a variable light chain polypeptide selected from VL of SEQ ID NO:18, SEQ ID NO:19, SEQ ID NO:20, SEQ ID NO:21 or SEQ ID NO:22.

In some of these embodiments, i is from 2 to 4.

In one embodiment, the antibody drug conjugate corresponds to the following formula (XIII) 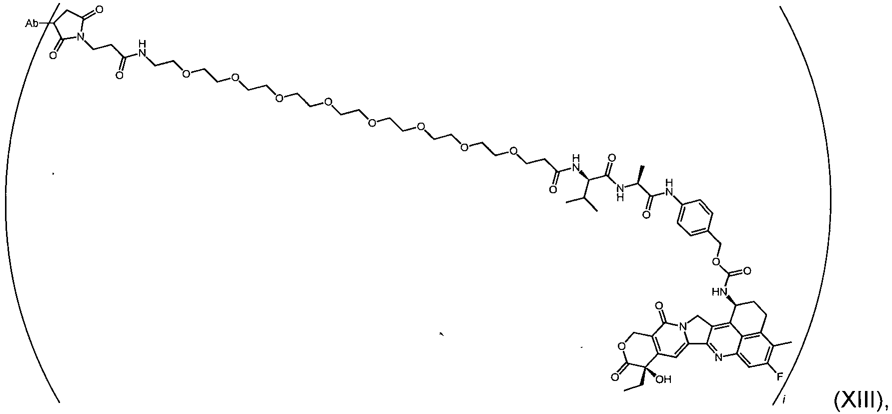 where i is from 1 to 8.

In some of these embodiments, Ab is an anti-CA242 antibody comprising a variable heavy chain polypeptide selected from VH of SEQ ID NO:13, SEQ ID NO:14, SEQ ID NO:15, SEQ ID NO:16 or SEQ ID NO:17 and comprising a variable light chain polypeptide selected from VL of SEQ ID NO:18, SEQ ID NO:19, SEQ ID NO:20, SEQ ID NO:21 or SEQ ID NO:22.

In some of these embodiments, *i* is from 2 to 4.

### Pharmaceutical compositions

In another aspect, the present disclosure provides a composition, *e.g.* a pharmaceutical composition, containing one or a combination of ADC disclosed herein, formulated together with a pharmaceutically acceptable carrier.

As used herein, "pharmaceutically acceptable carrier" includes any and all solvents, dispersion media, coatings, antibacterial and antifungal agents, isotonic and absorption delaying agents, and the like that are physiologically compatible. The pharmaceutical formulation of the disclosure may further comprise one or more pharmaceutically acceptable excipients selected from stabilizers, surfactants, buffering agents, antimicrobial preservatives, protectants, antioxidants, chelating agents, bulking agents.

### Treatments

As used herein, the term "treat" "treating" or "treatment" refers to one or more of (1) inhibiting the disease; for example, inhibiting a disease, condition or disorder in an individual who is experiencing or displaying the pathology or symptomatology of the disease, condition or disorder (i.e., arresting further development of the pathology and/or symptomatology); and (2) ameliorating the disease; for example, ameliorating a disease, condition or disorder in an individual who is experiencing or displaying the pathology or symptomatology of the disease, condition or disorder (i.e., reversing the pathology and/or symptomatology) such as decreasing the severity of disease or reducing or alleviating one or more symptoms of the disease. In particular, with reference to the treatment of a tumor, the term "treatment" may refer to the inhibition of the growth of the tumor, or the reduction of the size of the tumor.

As used herein, a "therapeutically efficient amount" or "effective amount" of an ADC is an amount sufficient to perform a specifically stated purpose, for example to produce a therapeutic effect after administration, such as inhibition or reduction in tumor growth rate or tumor volume, a reduction in a symptom of cancer or some indicia of treatment efficacy.

ADC of the present disclosure have therapeutic utilities. For example, these molecules can be administered in a subject, *e.g. in vivo,* to treat, or prevent a variety of disorders. It is contemplated herein to use the ADC of the present disclosure as a medicament, in particular for use in the treatment of cancer in a subject in need thereof, in particular cancer with tumor cells expressing CA242, more specifically, a cancer with a solid tumor, and more specifically selected from the group consisting of colorectal cancer, pancreatic cancer, gastric cancer and lung cancer.

The term "cancer" refers to or describe the physiological condition in mammals that is typically characterized by unregulated cell growth. Examples of cancer include, but are not limited to, carcinoma, lymphoma, blastoma, sarcoma, and leukemia. More particular examples of such cancers include squamous cell cancer, small-cell lung cancer, non-small cell lung cancer, adenocarcinoma of the lung, squamous carcinoma of the lung, cancer of the peritoneum, hepatocellular cancer, gastrointestinal cancer, pancreatic cancer, glioblastoma, cervical cancer, ovarian cancer, fallopian tube cancer, liver cancer, bladder cancer, hepatoma, breast cancer, colon cancer, colorectal cancer, endometrial or uterine carcinoma, salivary gland carcinoma, kidney cancer, liver cancer, prostate cancer, vulval cancer, thyroid cancer, hepatic carcinoma and various types of head and neck cancers.

The ADC of the disclosure are particularly useful in the treatment of cancer selected from the group consisting of colorectal cancer, pancreatic cancer, gastric cancer and lung cancer. Hence, the disclosure relates to a method of treating cancer, in particular one of the above listed cancers, and more preferably cancer selected from the group consisting of colorectal cancer, pancreatic cancer, gastric cancer and lung cancer, said method comprising administering a therapeutically efficient amount of an ADC of formula (I) as disclosed herein.

Embodiments will now be described by way of example and not limitation with reference to the accompanying figures.

### Brief description of Figures

The accompanying drawings illustrate exemplary embodiments of the disclosure, and together with the general description and the detailed description of the embodiments, explain, by way of example, the principles of the disclosure.
Figure 1 shows *in vitro* cell binding of new anti-CA242 humanized monoclonal antibodies on Colo-205 cell line.
Figure 2 shows *in vitro* cell internalization of new anti-CA242 humanized monoclonal antibodies on Colo-205 cell line. Figure 2A shows internalization at 37°C and Figure 2B at 4°C (negative control plate).
Figure 3A shows *in vivo* efficacy (tumor growth over time) of antibody-drug conjugates in HT-29 colorectal cancer model.
Figure 3B shows *in vivo* efficacy (tumor growth over time) of DL1-based antibody-drug conjugates in HT-29 colorectal cancer model.
Figure 3C shows *in vivo* efficacy (tumor growth over time) of DL1, DL3 and DL4-based antibody-drug conjugates in HT-29 colorectal cancer model.
Figure 3D shows *in vivo* efficacy (tumor growth over time) of DL1, DL3 and DL4-based antibody-drug conjugates and comparator cantuzumab-DM4 in HT-29 colorectal cancer model.
Figure 3E shows *in vivo* efficacy (tumor growth over time) of DL1, DL3 and DL4-based antibody-drug conjugates and comparator cantuzumab-DM4 in HT-29 colorectal cancer model.
Figure 3F shows *in vivo* efficacy (tumor growth over time) of DL1, DL3 and DL4-based antibody-drug conjugates and comparator cantuzumab-DM4 in HT-29 colorectal cancer model.

### Examples

### Example 1: generation of humanized anti-CA242 antibodies

The humanization process was performed using an in-silico method based on CDR-grafting onto a curated selection of human germline sequences, with additional non-CDR residue back-mutations based on structural models. Parental CDRs were identified according to the IMGT definition of CDRs. Murine anti-CA242 parental variable heavy (VH) and variable light (VL) used for humanization were obtained in patent US5552293A and are respectively disclosed in SEQ ID NO: 7 and SEQ ID NO: 8.

At the start of the humanization process, a homology model of the parental VH and VL chains is built in a single chain variable fragment (scFv) format. The modeling is done in four stages: gathering homologous sequences; fold library scanning; loop modeling and finally side chain placement. The resulting model is used to guide the choice of donor or acceptor amino acids during the humanization process.

The parental VH and VL sequences are aligned with a selected panel of human germline sequences. The closest matching germlines from 2 different VH and VL families are selected. A proprietary humanization algorithm is then used to select CDRs and framework amino acids to graft from the donor parental sequences onto the human acceptor germline sequence with 4 VH and 4 VL sequences produced leading to a possible 16 variants if expressed in combination. Humanized VH and VL sequences are disclosed in SEQ ID NO: 13-22.

The percentage identity value to humanness is calculated by aligning the corresponding humanized VH and VL to all human germlines by the IMGT domain gap alignment tool - (https://www.imgt.org/3Dstructure-DB/cgi/DomainGapAlign.cgi). The humanness score (T20 score) for the humanized VH and VL were calculated using the method described in Gao 2013.

The humanized heavy and light chains were combined to create 16 different variants, that were tested for their expression level, antigen-binding affinity and internalization properties in order to identify antibodies that perform similarly to the chimeric parental antibody and known antibody cantuzumab (as described in International Nonproprietary Names for Pharmaceutical Substances (INN) List 67, published in the World Health Organization (WHO) Drug Information Vol 26, No. 1, 2012). The tested antibodies were produced (50mL scale) in CHO K1 cells (WuXi Biologics WuXian Transient CHO expression system, 7 days incubation, protein A purification).

Produced antibodies are shown in Table 1 below. Humanness score of VH and VL sequences are presented in Table 2 below.

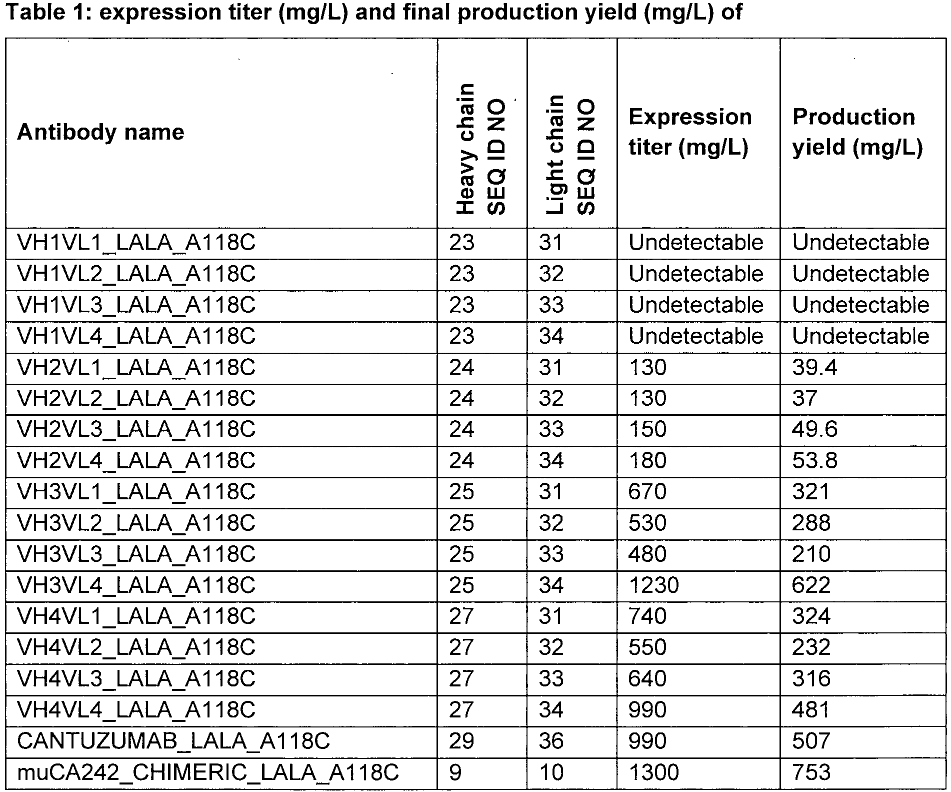

**Table 2: T20 humanness score of VH and VL**

| **Variable region** | **SEQ ID NO:** | **T20 humanness score** |
|---|---|---|
| VH1 | 13 | 78.7 |
| VH2 | 14 | 89.7 |
| VH3 | 15 | 87.2 |
| VH4 | 16 | 91.6 |
| VL1 | 18 | 59.4 |
| VL2 | 19 | 59.4 |
| VL3 | 20 | 61.5 |
| VL4 | 21 | 61.5 |
| CANTUZUMAB VH | 17 | 75.9 |
| CANTUZUMAB VL | 22 | 57.6 |

Expression titers of the VH1 and VH2-based antibodies were deemed too low and unsatisfactory for further development and were not further investigated. Humanness scores of the different VH and VL sequences were deemed acceptable.

### Example 2: in vitro cell binding assay on Colo-205 cell line by flow cytometry

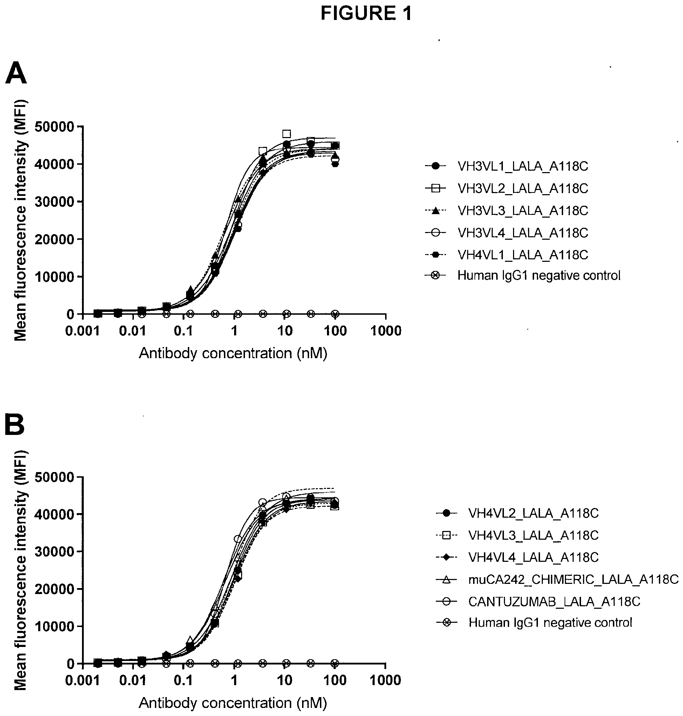

The cell binding affinity of antibodies was assessed by flow cytometry on CA242 positive cancer cell line Colo-205. 1×10⁵ cells per well were aliquoted in a 96-well plate and washed once with 1% FBS in PBS (assay buffer). Humanized antibodies, reference chimeric antibody and isotype control human IgG1 antibody (produced by WuXi Biologics) were diluted with assay buffer (11 concentrations starting at 100 nM with 3-fold dilutions). 100 µL was added to each well. Cells were incubated at 4°C for 1 hour followed by washing with assay buffer twice. 100µL of a solution of secondary detection goat antibody PE-conjugated anti-human IgG (Fc) (Jackson ImmunoResearch cat#109-115-098, diluted 1:150 with assay buffer) was added into each well. Cells were incubated at 4°C for 0.5 hours and cells were re-suspended in 200µL of assay buffer for flow cytometry analysis on a BD FACSCanto II instrument. Mean Fluorescence Intensity (MFI) was used to calculate EC₅₀. Results are presented in Table 3 below and in Figures 1A and 1B (divided for clarity purposes).

**Table 3: EC₅₀ cell binding values of the antibodies, as assessed by flow cytometry on CA242 positive Colo-205 cell line**

| **Antibody name** | **Binding EC₅₀ (nM)** |
|---|---|
| VH3VL1_LALA_A118C | 0.91 |
| VH3VL2_LALA_A118C | 0.94 |
| VH3VL3_LALA_A118C | 0.63 |
| VH3VL4_LALA_A118C | 0.97 |
| VH4VL1_LALA_A118C | 1.01 |
| VH4VL2_LALA_A118C | 0.96 |
| VH4VL3_LALA_A118C | 1.00 |
| VH4VL4_LALA_A118C | 1.07 |
| muCA242_CHIMERIC_LALA_A 118C | 0.70 |
| CANTUZUMAB_LALA_A118C | 0.68 |

All humanized antibodies exhibited comparable and acceptable binding affinity.

### Example 3: in vitro cell internalization assay on Colo-205 cell line by flow cytometry

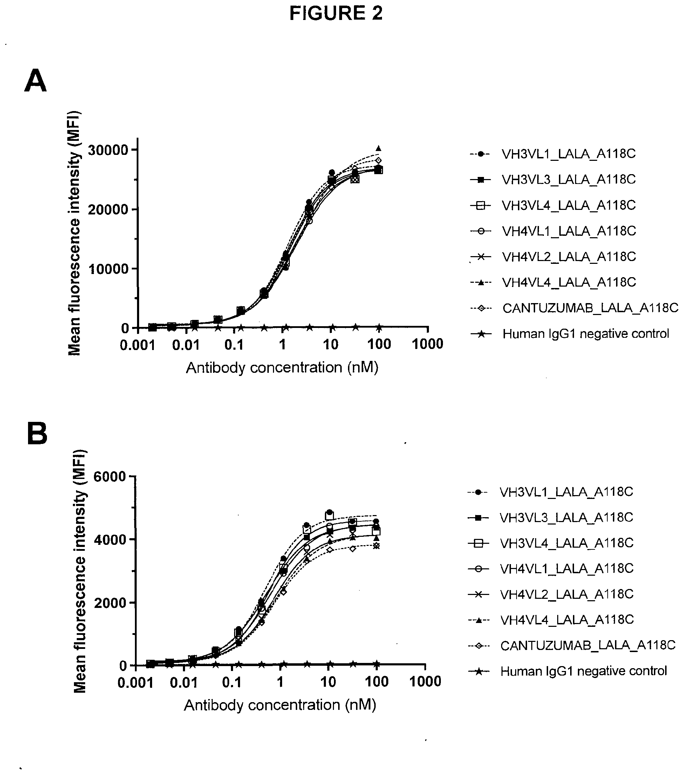

The cell internalization capability of antibodies was assessed by flow cytometry on CA242 positive cancer cell line Colo-205. 1.5×10⁵ cells per well were aliquoted in a 96-well plate and washed once with 1% FBS in PBS (assay buffer). Humanized antibodies and isotype control human IgG1 antibody (produced by WuXi Biologics) were diluted with assay buffer (11 concentrations starting at 100 nM with 3-fold dilutions). 100 µL was added to each well. Cells were incubated at 4°C for 1 hour followed by washing with assay buffer twice. 150µL of a solution of secondary detection goat antibody PE-conjugated anti-human IgG (Fc) (Jackson ImmunoResearch cat#109-115-098, diluted 1:150 with assay buffer) was added into each well. Cells were incubated at either 37°C (internalization plate) or 4°C (control plate) for 3 hours. 100µL per well of glycine hydrochloride buffer (pH 2.5) was added at 4°C during 4 minutes to quench antibody binding interactions and release cell-surface bound primary and PE-conjugated secondary antibodies. Cells were then re-suspended in 200µL of assay buffer for flow cytometry analysis on a BD FACSCanto II instrument. Mean Fluorescence Intensity (MFI) was used to calculate EC₅₀. Results are presented in Table 4 below and in Figure 2A (internalization plate at 37°C) and Figure 2B (control plate at +4°C).

**Table 4: EC₅₀ cell internalization values of the antibodies, as assessed by flow cytometry on CA242 positive Colo-205 cell line**

| **Incubation conditions** | **Antibody name** | **Internalization EC₅₀ (nM)** | **Max MFI** |
|---|---|---|---|
| 37°C 3h | VH3VL1_LALA_A118C | 1.34 | 26800 |
| | VH3VL3_LALA_A118C | 1.42 | 26300 |
| | VH3VL4_LALA_A118C | 1.54 | 26400 |
| | VH4VL1_LALA_A118C | 1.95 | 26800 |
| | VH4VL2_LALA_A118C | 1.78 | 26700 |
| | VH4VL4_LALA_A118C | 2.30 | 30100 |
| | CANTUZUMAB _LALA_A118C | 1.69 | 28000 |
| | HUMAN IGG1 NEG CONTROL | N/A | 37.9 |
| 4°C 3h | VH3VL1_LALA_A118C | 0.51 | 4835 |
| | VH3VL3_LALA_A118C | 0.53 | 4342 |
| | VH3VL4_LALA_A118C | 0.61 | 4715 |
| | VH4VL1_LALA_A118C | 0.64 | 4395 |
| | VH4VL2_LALA_A118C | 0.76 | 4158 |
| | VH4VL4_LALA_A118C | 0.84 | 4033 |
| | CANTUZUMAB_LALA_A118C | 0.77 | 3770 |
| | HUMAN IGG1 NEG CONTROL | N/A | 33 |

All humanized antibodies exhibited comparable and acceptable internalization capabilities.

### Example 4: Synthesis of drug-linker compounds

### General

The following materials and synthesis methods are applicable to the synthetic procedures described in this section unless indicated otherwise. All commercially available anhydrous solvents were used without further purification. Starting materials, reagents, active pharmaceutical ingredients and solvents were purchased from reputable commercial suppliers (Sigma-Aldrich, Fisher Scientific, MedChemExpress, TCI Chemicals, Broadpharm, WuXi LabNetwork, WuXi STA, Enamine, Fluorochem) and used without further purification unless stated otherwise.

Normal phase flash chromatography was performed on Teledyne Isco CombiFlash^{®} devices using silica flash cartridges (40-63µm particle size). Preparative HPLC was performed on a AUNO LC-2000 system equipped with a binary pump system and UV detection (220 and 254nm). Mobile phase A was water + 0.1% TFA and mobile phase B was acetonitrile. Column was a Welch Ultimate XB-C18 50x250mm 7µm maintained at room temperature. Injection volumes were 3-10mL. Generic linear gradient was typically 20%B to 60%B in 45 min + hold at 60%B for 5 min. Flow rate was 60 mL/min.

It will be appreciated that, in the description of the synthetic methods described below, all proposed reaction conditions, including the choice of solvent, organic base, coupling reagent, reaction temperature, duration of the experiment or any workup procedures employed, can be selected or modified by a person skilled in the art of organic synthesis. It will also be understood by one skilled in the art of organic synthesis that the functionality present on various portions of the molecule must be compatible with the reagents and reaction conditions utilized.

### Analytical methods

HPLC-DAD Method 1: Shimadzu LC-20AD system equipped with DAD detection. Mobile phase A was water + 0.04% TFA and mobile phase B was acetonitrile + 0.02% TFA. Column was a Phenomenex Kinetex C18 4.6x50mm 5µm (maintained at 50°C). Linear gradient was 10%B to 80%B in 5 min + hold at 80%B for 0.70 min. Flow rate was 1.5 mL/min. UHPLC-QTOF Method 2: Agilent 1290 UHPLC system equipped with Agilent G6545B QTOF high resolution mass spectrometry system. Mobile phase A was water + 0.1% formic acid and mobile phase B was acetonitrile. Column was an Agilent Poroshell EC-C18 2.1x50mm 1.9µm (maintained at 40°C). Linear gradient was 1%B to 100%B in 3 min. Flow rate was 1.0 mL/min. Mass spectrometer was used in ESI mode (Agilent Dual AJG ESI source), Nebulized was 35 psi, N₂ Drying Gas Flow was 8 L/min, Gas Temp and Sheath Gas Temp were 320°C, Capillary Voltage was 3500 V (positive mode), Mass Range was 100-3200 m/z. Liquid nuclear magnetic resonance (NMR) spectra were recorded on a Bruker AVANCE III HD400 spectrometer, using residual solvent peak for calibration.

### Synthesis of DL1

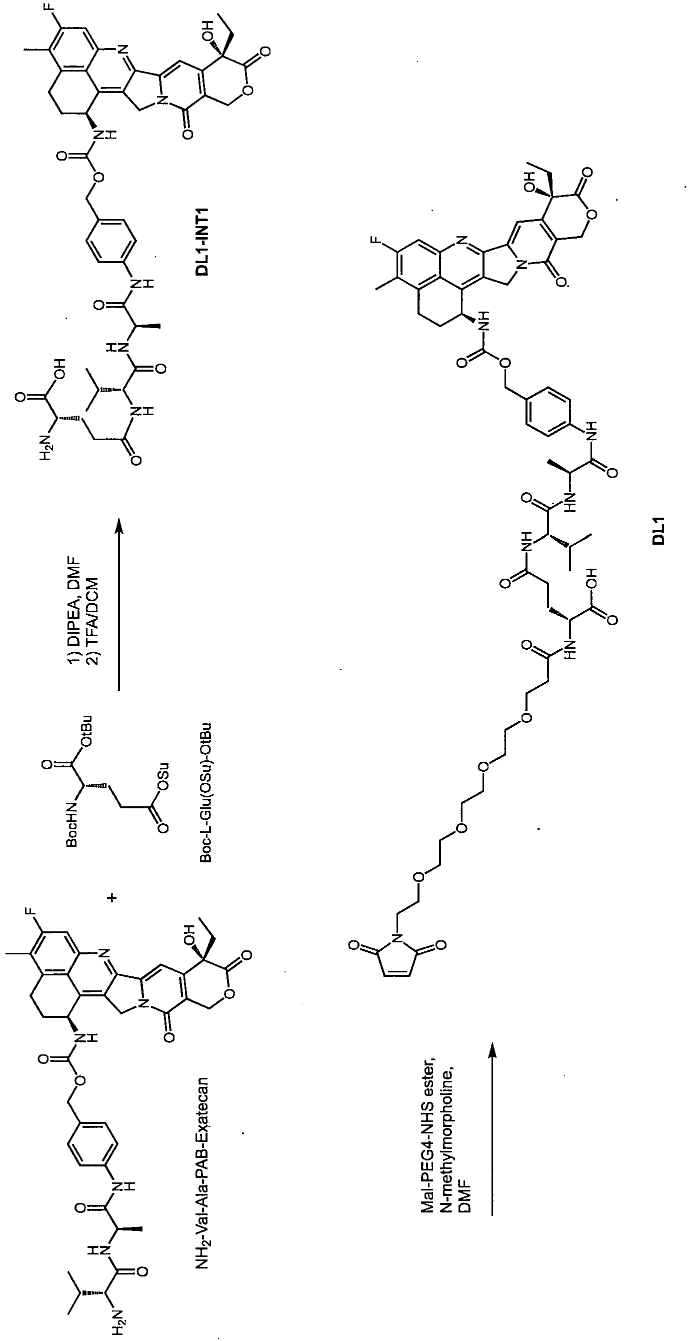

Commercially available NH₂-Val-Ala-PAB-Exatecan (CAS#2845164-91-0, 1 molar eq) and commercially available Boc-L-Glu(Osu)-OtBu (CAS#81659-82-7, 1.3 molar eq) were dissolved in anhydrous DMF. Diisopropylethylamine (2 molar eq) was added, and the mixture was stirred at room temperature for 2 hours. The reaction was diluted with saturated NaHCOs solution and extracted 3 times with DCM. The organic phase was dried over MgSO₄, filtered and evaporated under vacuum to afford intermediate compound as crude brown solid. It was then dissolved on ice with TFA/DCM (30:70 v/v) and stirred at 0°C for 4 hours. Evaporation under vacuum and trituration with diethyl ether afforded a crude brown solid, that was further purified by preparative HPLC on C18 column (generic gradient from 20 to 90% acetonitrile in water, 0.05% TFA as modifier). Intermediate compound **DL1-INT1** was obtained as a yellow solid (77.8mg, 35-50% yield). HPLC purity was above 98% by HPLC on C18 column at 254nm.

This intermediate compound (1 molar eq) and commercially available Mal-PEG4-NHS ester (CAS#1325208-25-0, 1 molar eq) were dissolved in anhydrous DMF. 4-methylmorpholine (2 molar eq) was added, and the mixture was stirred at room temperature for 2 hours. Reaction was diluted with 0.1% TFA in water and directly purified by preparative HPLC on C18 column (generic gradient from 20 to 90% acetonitrile in water, 0.05% TFA as modifier). This afforded 63.9mg of final compound **DL1** as a yellow solid. HPLC method 1 purity was 96.2%. HPLC method 1 retention time was 2.62min. HRMS *m*/*z* (ESI⁺): Calc [M+H]⁺ = 1211.4942 ; Exp [M+H]⁺ = 1211.4929 ; Error = -1.3 ppm. ¹H NMR (400 MHz, DMSO-*d*₆) 11.76 - 13.28 (m, 1 H), 9.93 (s, 1 H), 8.17 - 8.23 (m, 1 H), 8.08 (br dd, *J*=19.51, 8.13 Hz, 2 H), 7.85 (br d, *J*=8.63 Hz, 1 H), 7.77 (br d, *J*=10.51 Hz, 1 H), 7.60 (br d, *J*=8.38 Hz, 2 H), 7.36 (br d, *J*=8.50 Hz, 2 H), 7.31 (s, 1 H), 7.01 (s, 1 H), 6.48 - 6.59 (m, 1 H), 5.45 (s, 2 H), 5.22 - 5.35 (m, 3 H), 5.07 (s, 2 H), 4.37 (quin, *J*=6.97 Hz, 1 H), 4.12 - 4.24 (m, 2 H), 3.52 - 3.62 (m, 6 H), 3.42 - 3.49 (m, 11 H), 3.17 - 3.30 (m, 2 H), 3.06 - 3.17 (m, 1 H), 2.34 - 2.44 (m, 5 H), 2.13 - 2.31 (m, 4 H), 1.82 - 2.00 (m, 4 H), 1.69 - 1.80 (m, 1 H), 1.30 (br d, *J*=7.13 Hz, 3 H), 0.80 - 0.93 (m, 9 H).

### Synthesis of DL2

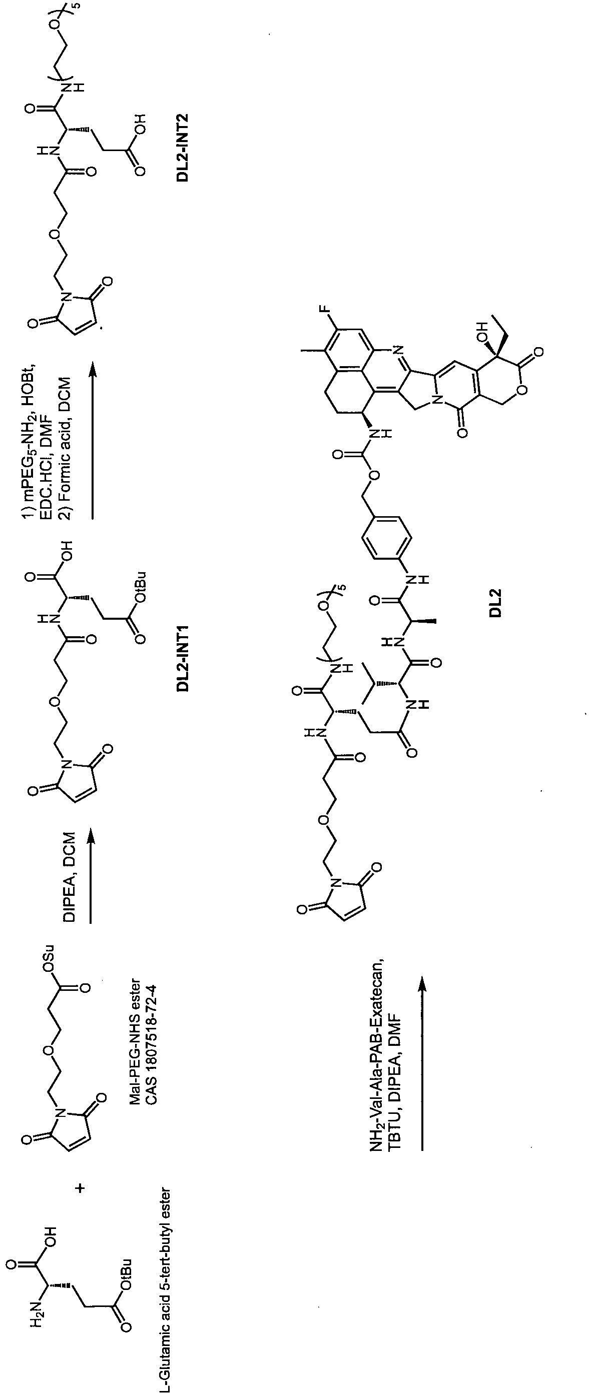

Commercially available Mal-PEG-NHS ester (CAS#1807518-72-4, 1 molar eq) and L-Glutamic acid 5-tert-butyl ester (1.14 molar eq) were dissolved in anhydrous DCM. Diisopropylethylamine (2 molar eq) was added, and the mixture was stirred at room temperature for 1 hour. The reaction was diluted with saturated citric acid solution and extracted 3 times with DCM. The organic phase was dried over MgSO₄, filtered and evaporated under vacuum to afford 2.1g (75% yield) of intermediate compound **DL2-INT1** as a crude yellow solid after trituration with diethyl ether. HPLC method 1 purity was 97.4%. This compound was engaged into next reaction without further characterization or purification.

mPEG₅-NH₂ (CAS# 5498-83-9, 1 molar eq), HOBt (1 molar eq), compound DL2-INT1 (1 molar eq) and DIPEA (2 molar eq) were dissolved in anhydrous DMF on ice. EDC.HCl (1.2 molar eq) was added, the reaction mixture was allowed to reach room temperature, and the reaction was stirred at room temperature for 1 hour. Evaporation under vacuum and trituration with diethyl ether afforded a crude brown mass that was dissolved in formic acid/DCM (80:20 v/v) for tert-butyl ester deprotection. Reaction was stirred for 4 hours at room temperature. Evaporation under vacuum and trituration with diethyl ether afforded a crude brown solid that was purified by chromatography on silica gel (DCM/MeOH, gradient from 100:0 to 85:15) to afford 135mg (20% yield) of compound **DL2-INT2** as a slightly yellow solid. HPLC method 1 purity was 94.1%. This compound was engaged into next reaction without further characterization or purification.

**DL2-INT2** (1 molar eq) and TBTU (1 molar eq) were dissolved in anhydrous DMF. DIPEA (1.5 molar eq) was added and the reaction was stirred for 10min at room temperature. Commercially available NH₂-Val-Ala-Exatecan (CAS#2845164-91-0, 1 molar eq) was added and the reaction was stirred at room temperature for 1 hour. The reaction was diluted with saturated citric acid solution and extracted 3 times with DCM. The organic phase was dried over MgSO₄, filtered and evaporated under vacuum to afford a crude yellow intermediate solid after trituration with diethyl ether. Purification by preparative HPLC on C18 column (generic gradient from 20 to 90% acetonitrile in water, 0.05% TFA as modifier) afforded 57.5mg (50% yield) of final compound **DL2** as a yellow solid. HPLC method 1 purity was 98.2%. HPLC method 1 retention time was 2.76min. HRMS *m*/*z* (ESI⁺): Calc [M+H]⁺ = 1312.5783 ; Exp [M+H]⁺ = 1312.5770; Error = -1.3 ppm. ¹H NMR (400 MHz, DMSO-*d*₆) 9.93 (s, 1 H), 8.20 (br d, *J*=6.75 Hz, 1 H), 8.05 (br d, *J*=8.76 Hz, 1 H), 7.95 (br d, *J*=8.00 Hz, 1 H), 7.88 (br t, *J*=5.50 Hz, 1 H), 7.73 - 7.83 (m, 2 H), 7.59 (br d, *J*=8.38 Hz, 2 H), 7.36 (br d, *J*=8.38 Hz, 2 H), 7.31 (s, 1 H), 7.00 (s, 1 H), 6.52 (br s, 1 H), 5.45 (s, 2 H), 5.23 - 5.35 (m, 3 H), 5.07 (s, 2 H), 4.38 (quin, *J*=6.97 Hz, 1 H), 4.13 - 4.26 (m, 2 H), 3.53 (br dd, *J*=12.38, 6.88 Hz, 8 H), 3.49 (s, 11 H), 3.40 - 3.43 (m, 4 H), 3.02 - 3.30 (m, 8 H), 2.30 - 2.39 (m, 5 H), 2.13 - 2.26 (m, 4 H), 1.88 - 2.00 (m, 2 H), 1.78 - 1.88 (m, 2 H), 1.62 - 1.76 (m, 1 H), 1.30 (br d, *J*=7.00 Hz, 3 H), 0.81 - 0.92 (m, 9 H).

### Procedure for the synthesis of DL3

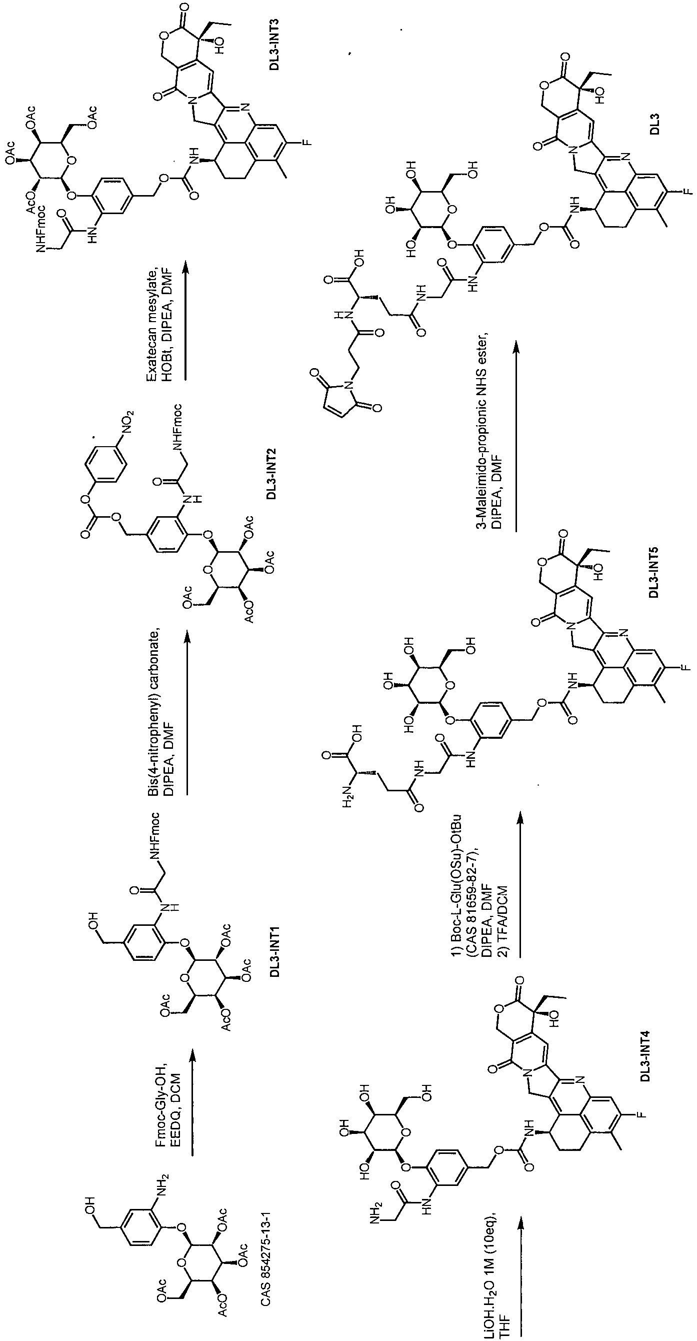

(2R,3S,4S,5R,6S)-2-(acetoxymethyl)-6-(2-amino-4-(hydroxymethyl)phenoxy)tetrahydro-2H-pyran-3,4,5-triyl triacetate (CAS#854275-13-1, 1 molar eq, commercially available or synthesized as described in Lilley 2020), Fmoc-Gly-OH (1 molar eq) and EEDQ (2 molar eq) were dissolved in anhydrous DCM. Reaction was stirred at room temperature for 1 hour, was diluted with saturated citric acid solution and extracted 3 times with DCM. The organic phase was dried over MgSO₄, filtered and evaporated under vacuum. Crude product was purified by chromatography on silica gel (petroleum ether/EtOAc, gradient from 90:10 to 10:90) to afford 2.4g (72% yield) of compound **DL3-INT1** as a white solid. HPLC method 1 purity was above 95%. This compound was engaged into next reaction without further characterization.

**DL3-INT1** (1 molar eq) and Bis(4-nitrophenyl) carbonate (3 molar eq) were dissolved in anhydrous DMF, and DIPEA (6 molar eq) was added. Reaction was stirred at room temperature for 3 hours, was diluted with saturated citric acid solution and extracted 3 times with EtOAc. The organic phase was dried over MgSO₄, filtered and evaporated under vacuum to afford a crude that was purified by chromatography on silica gel (petroleum ether/EtOAc, gradient from 90:10 to 10:90) to afford 3.72g (76% yield) of compound **DL3-INT2** as a slightly yellow solid. HPLC method 1 purity was 84.6%. This compound was engaged into next reaction without further characterization or purification.

**DL3-INT2** (1 molar eq), exatecan mesylate (0.8 molar eq) and HOBt (1.2 molar eq) were dissolved in anhydrous DMF, and DIPEA (6 molar eq) was added. Reaction was stirred at room temperature for 3 hours, was diluted with saturated citric acid solution and extracted 3 times with DCM. The organic phase was dried over MgSO₄, filtered and evaporated under vacuum to afford a crude that was purified by chromatography on silica gel (DCM/MeOH, gradient from 100:0 to 95:5) to afford 1.49g (72% yield) of compound **DL3-INT3** as a yellow solid. HPLC method 1 purity was 85.3%. This compound was engaged into next reaction without further characterization or purification.

**DL3-INT3** (1 molar eq) was dissolved in THF at 0°C and 10 molar equivalent of lithium hydroxide (from a cold 1M aqueous solution) were added into the reaction vessel. The reaction was stirred for 2 hours and quenched with 20 molar equivalents of cold acetic acid. Purification of the reaction mixture by preparative HPLC on C18 column (generic gradient from 20 to 90% acetonitrile in water, 0.05% TFA as modifier) afforded 311mg (20% yield) of intermediate compound **DL3-INT4** as a yellow solid. HPLC method 1 purity was 98.5%. This compound was engaged into next reaction without further characterization.

Commercially available Boc-L-Glu(Osu)-OtBu (CAS#81659-82-7, 1 molar eq) and **DL3-INT4** (1 molar eq) were dissolved in DMF, and DIPEA (2 molar eq) was added. Reaction was stirred at room temperature for 2 hours. After removal of volatiles under vacuum, residue was diluted with TFA/DCM (50:50 v/v) and stirred for 4 hours at room temperature. When Boc and tert-butyl ester deprotection were completed, as observed by HPLC, volatiles were removed under reduced pressure and crude residue was purified using preparative HPLC on C18 column (generic gradient from 20 to 90% acetonitrile in water, 0.05% TFA as modifier) to afford 47mg (38% yield) of compound **DL3-INT5** as a yellow solid. HPLC method 1 purity was 95.5%. This compound was engaged into next reaction without further characterization.

3-maleimido-propionic acid ester (1.5 molar eq) and **DL3-INT5** (1 molar eq) were dissolved in anhydrous DMF. DIPEA (2 molar eq) was added, and the mixture was stirred at room temperature for 1 hour. Reaction was diluted with 0.1% TFA in water and directly purified by preparative HPLC on C18 column (generic gradient from 20 to 90% acetonitrile in water, 0.05% TFA as modifier). This afforded 52mg (86% yield) of final compound **DL3** as a yellow solid. HPLC method 1 purity was 99.0%. HPLC method 1 retention time was 2.00min. HRMS *m*/*z* (ESI⁺): Calc [M+H]⁺ = 1100.3532 ; Exp [M+H]⁺ = 1100.3527; Error = -0.5 ppm. ¹H NMR (400 MHz, DMSO-*d*₆) 12.01 - 13.07 (m, 1 H), 9.16 (s, 1 H), 8.26 (d, *J*=7.75 Hz, 1 H), 8.21 (br t, *J*=5.75 Hz, 1 H), 8.15 - 8.19 (m, 1 H), 8.15 - 8.19 (m, 1 H), 8.05 (br d, *J*=8.63 Hz, 1 H), 7.77 (d, *J*=10.88 Hz, 1 H), 7.30 (s, 1 H), 7.19 (d, *J*=8.38 Hz, 1 H), 7.09 (br d, *J*=8.25 Hz, 1 H), 6.99 (s, 1 H), 6.51 (br s, 1 H), 5.49 - 5.64 (m, 1 H), 5.43 (s, 2 H), 5.26 (s, 3 H), 4.98 - 5.14 (m, 2 H), 4.63 (br d, *J*=7.75 Hz, 1 H), 4.13 (td, *J*=8.22, 5.19 Hz, 1 H), 3.82 - 3.97 (m, 2 H), 3.71 (br d, *J*=3.13 Hz, 1 H), 3.66 (br d, *J*=8.13 Hz, 1 H), 3.57 - 3.63 (m, 3 H), 3.55 (br d, *J*=3.25 Hz, 2 H), 3.52 (br s, 1 H), 3.45 (br d, *J*=3.13 Hz, 1 H), 3.42 (br d, *J*=3.13 Hz, 2 H), 3.19 - 3.28 (m, 2 H), 3.07 - 3.15 (m, 1 H), 2.34 - 2.45 (m, 5 H), 2.13 - 2.29 (m, 4 H), 1.74 - 2.01 (m, 4 H), 0.88 (t, *J*=7.32 Hz, 2 H), 0.82 - 0.93 (m, 1 H).

### DL4

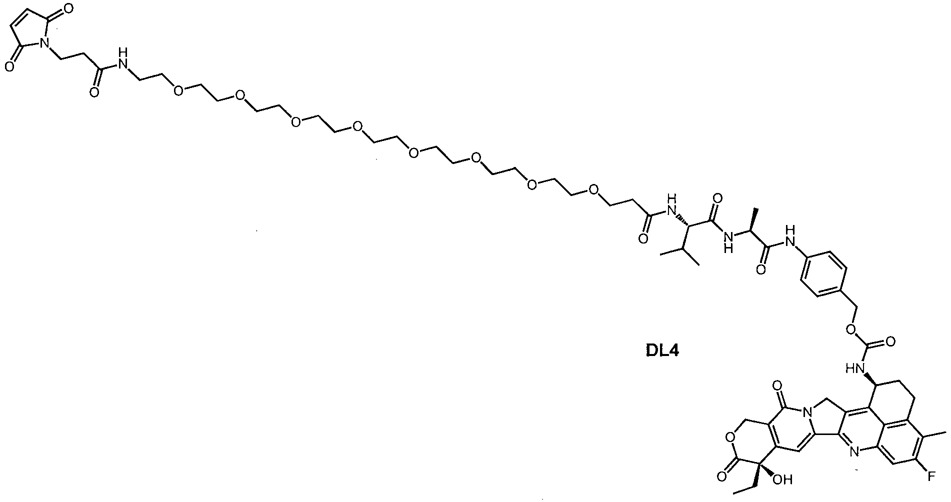

DL4 was purchased from MedChemExpress (cat#HY-147271) and was used without prior analytical characterizations.

### Example 5: Synthesis of antibody-drug conjugates (ADCs)

### Antibody production

Monoclonal antibodies were produced by transient transfection of CHO K1 cells using art-recognized techniques (WuXi Biologics). cDNAs were cloned into proprietary vector system using conventional cloning techniques. Suspension-adapted CHO K1 cells were used for antibody production. The seed was grown in WuXi Biologics' proprietary animal-free component medium, and supernatant was harvested by centrifugation and subsequent filtration (0.2µm filter). The antibodies were purified using protein A purification resin and SEC preparative resin. Purity of antibody materials was confirmed by reduced/non-reduced SDS-PAGE and size exclusion chromatography. Monomeric purity of monoclonal antibodies was >95%.

**Table 2A: overview of the synthesized monoclonal antibodies**

| **Antibody name** | **Heavy chain SEQ ID NO:** | **Light chain SEQ ID NO:** |
|---|---|---|
| VH1VL1_LALA_A118C | 23 | 31 |
| VH1VL2_LALA_A118C | 23 | 32 |
| VH1VL3_LALA_A118C | 23 | 33 |
| VH1VL4_LALA_A118C | 23 | 34 |
| VH2VL1_LALA_A118C | 24 | 31 |
| VH2VL2_LALA_A118C | 24 | 32 |
| VH2VL3_LALA_A118C | 24 | 33 |
| VH2VL4_LALA_A118C | 24 | 34 |
| VH3VL1_LALA_A118C | 25 | 31 |
| VH3VL2_LALA_A118C | 25 | 32 |
| VH3VL3_LALA_A118C | 25 | 33 |
| VH3VL4_LALA_A118C | 25 | 34 |
| VH4VL1_LALA_A118C | 27 | 31 |
| VH4VL2 _LALA_A118C | 27 | 32 |
| VH4VL3_LALA_A118C | 27 | 33 |
| VH4VL4_LALA_A1180 | 27 | 34 |
| CANTUZUMAB_LALA_A118C | 29 | 36 |
| muCA242_CHIMERIC_LALA_A118C | 9 | 10 |
| muCA242_lgG2a | 11 | 12 |
| VH3VL4_LALA_V205C | 26 | 35 |
| VH3VL4_LALA_V205C_A1180 | 25 | 35 |
| VH4VL4_LALA_V205C | 28 | 35 |
| VH4VL4_LALA_V205C_A118C | 27 | 35 |
| CANTUZUMAB_LALA_V205C | 30 | 37 |
| CANTUZUMAB_LALA_V205C_A118C | 29 | 37 |
| NEGIGG_LALA_V205C | 39 | 38 |
| NEGIGG_LALA_V205C_A118C | 40 | 38 |

### Preparation of site-specific cysteine conjugated antibody-drug conjugates (bioconjugation method 1)

pH of the monoclonal antibody solution (concentration 10-20mg/mL; 20mM histidine buffer pH 6.0) was adjusted to pH 8.0 by adding 1M Tris buffer. Ethylenediaminetetraacetic acid (EDTA) was added for a final concentration of 2mM. Antibody was fully reduced at room temperature overnight by adding a 50-100 molar excess of dithiothreitol (DTT). Antibody was then purified by Cation Exchange Chromatography (CEX) and inter-chain cysteines were then re-oxidized at room temperature for 1.5 hours by adding a 15-30 molar excess of dehydroascorbic acid (dHAA). Antibody was then purified by Cation Exchange Chromatography (CEX) and concentrated using Amicon 30K centrifugal filter devices (Millipore). EDTA was added for a final concentration of 2mM, followed by sterile filtration with a 0.22 µm filter and storage of the antibody at -80°C until bioconjugation. Antibody was characterized by UV absorbance at 280nm for concentration, endotoxin level determination, LC-QTOF and HPLC-SEC.

Before bioconjugation and if necessary, 1M Tris buffer was added into the antibody solution (5-15 mg/mL) to adjust pH to ~6.5. Drug-linker was added, from a 10mM dimethylacetamide (DMA) stock solution. 2.75 molar equivalents of drug-linker were used for DAR2 ADCs and 6 molar equivalents of drug-linker were used for DAR4 ADCs. DMA co-solvent adjusted to reach a final 10% v/v. Bioconjugation was performed at room temperature for 1 hour (monitoring advancement of the reaction by LC-TOF). ADCs were then purified using Zeba 40K Spin Desalting columns (Thermo Scientific) and were buffer exchanged using Amicon 30K centrifugal filter devices. Final ADC buffer was 20mM histidine + 8% (w/v) sucrose pH 6.0. Final ADC products were sterile filtered through a 0.22 µm filter membrane, aliquoted and stored at -80°C. Final characterizations included ADC concentration by BCA assay (Pierce^{™} BCA Protein Assay Kit, Thermo Scientific), DAR assessment by reducing LC-TOF, monomeric purity by HPLC-SEC, RP-HPLC for free drug-linker content and endotoxin content (LAL Kinetic Turbidimetric Assay).

### Preparation of site-specific cysteine and site-specific hinge cysteine conjugated antibody-drug conjugates (bioconjugation method 2)

Monoclonal antibody solution was buffer exchanged with PBS pH 7.4 and antibody concentration was adjusted to 5-10 mg/mL. Antibody was then reduced using 8 molar eq of TCEP for 1.5 hours at room temp..1.4 molar eq of Zn(OAc)₂ was then added (from a 1mM water solution) and antibody was incubated 20 min at room temperature. Hinge cysteines where then re-oxidized at room temperature for 1 hour by adding a 15 molar excess of dHAA (from a 10mM DMSO solution). 10 molar equivalent of drug-linker was added, from a 10mM DMA stock solution. DMA co-solvent adjusted to reach a final 10% v/v. 600 molar equivalent of EDTA was immediately added and bioconjugation was performed at 4°C for 1.5 hours (monitoring advancement of the reaction by LC-TOF). ADCs were then purified using Zeba 40K Spin Desalting columns (Thermo Scientific) and were buffer exchanged using Amicon 30K centrifugal filter devices. Final ADC buffer was 20mM histidine + 8% (w/v) sucrose pH 6.0. Final ADC products were sterile filtered through a 0.22 µm filter membrane, aliquoted and stored at -80°C. Final characterizations included ADC concentration by BCA assay (Pierce^{™} BCA Protein Assay Kit, Thermo Scientific), DAR assessment by reducing LC-TOF, monomeric purity by HPLC-SEC, RP-HPLC for free drug-linker content and endotoxin content (LAL Kinetic Turbidimetric Assay).

### Characterization of antibody-drug conjugates

### Drug-Antibody-Ratio (DAR) assessment by liquid chromatography-high resolution mass spectrometry (LC-TOF)

Final DAR was assessed by reversed phase liquid chromatography ESI+ high resolution TOF mass spectrometry, using an Agilent 1260 series HPLC system coupled to an Agilent 6224 TOF mass spectrometer. Conjugates were eluted on an Agilent PLRP-S 1000Å 2.1x50mm 8µm column maintained at 80°C, using a mobile phase gradient of water/acetonitrile (containing 0.025% TFA and 0.1% formic acid as modifiers) using a flow rate of 0.5mL/min. Data were analyzed using Agilent MassHunter Qualitative Analysis Software B.07.00. The reduced DAR value was calculated based on the peak abundance of the deconvoluted spectrum (addition of light chain and heavy chain DAR values).

### Monomeric purity by size exclusion chromatography (SEC)

SEC analysis was performed on an Agilent 1260 series HPLC system using a Tosoh TSKGEL G3000SWXL (7.8 × 300mm, 5µm) column maintained at room temperature. Mobile phase was 78 mM KH₂PO₄, 122 mM K₂HPO₄, 250 mM KCI, 15% isopropanol at pH 7.0. Flow rate was 0.75 mL/min. Sample loading was ~40-50 µg per injection. UV detection was monitored at 280 nm or any other relevant wavelengths.

### Free drug-linker content ("free payload")

Residual free drug-linker content was determined by HPLC-UV, using an Agilent 1260 HPLC system equipped with an Agilent Poroshell 120 SB-C18, 4.6 × 100 mm, 2.7 µm column maintained at 25°C. Mobile phase A was water + 0.05% TFA and mobile phase B was acetonitrile + 0.05% TFA. A generic linear gradient from 10%B to 80%B in 25 min was used. Flow rate was 0.6 mL/min. UV detection was performed at the camptothecin-specific absorption wavelength of 370 nm.

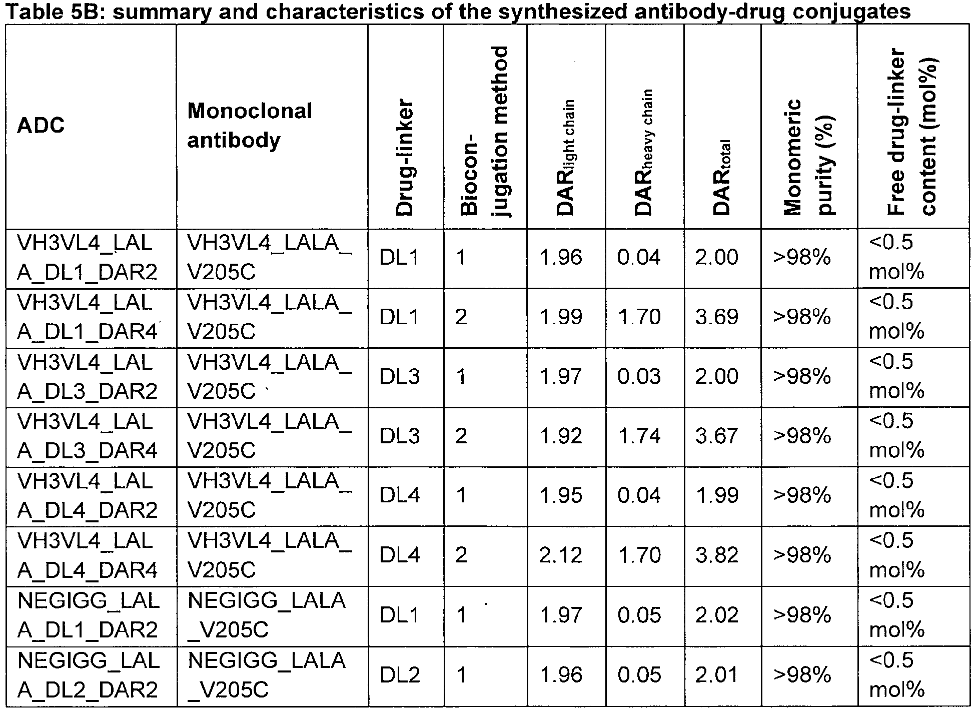

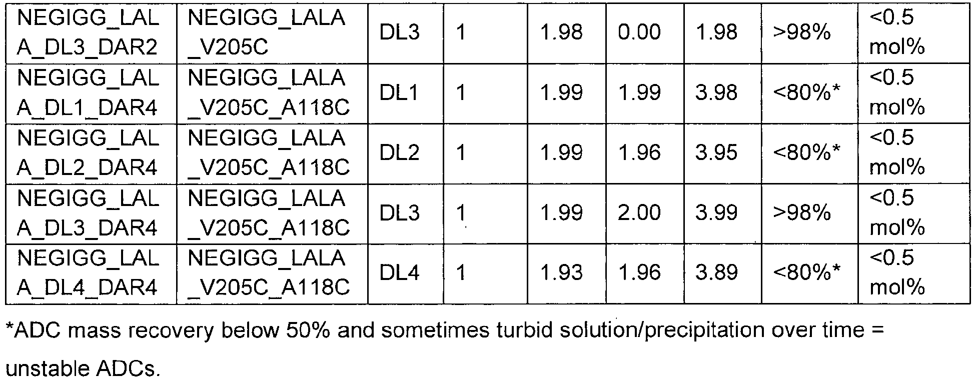

### Example 6: In vivo efficacy study in colorectal cancer model HT-29

Animals were maintained in a specific pathogen-free environment in polycarbonate cages (less than 5 mice per cage). All cages, bedding, and water were sterilized before use. Food and water were changed twice a week. During the study, the care and use of animals was conducted in accordance with the regulations of the Association for Assessment and Accreditation of Laboratory Animal Care (AAALAC). After tumor transplantation, the animals were checked daily for morbidity and mortality. At the time of routine monitoring, the animals were checked for any effects of tumor growth and treatments on normal behavior such as mobility, visual estimation of food and water consumption, body weight gain/loss (daily measurements), eye/hair matting and any other abnormal effect.

Six to eight-weeks-old immunocompromised female balb/c nude mice were inoculated subcutaneously with 3 × 10⁶ HT-29 cells resuspended in PBS/BD Matrigel (1:1 v/v). When average tumor volume reached 150 mm³ mice were randomized and treated by a single (unless stated otherwise) tail vein intravenous injection of saline (negative control) or investigated antibody-drug conjugate (diluted with saline before injection). Dosing volume was 5mL/kg. Tumor volumes were measured every 2-3 days in two dimensions using a caliper device (length × width) and calculated using the following formula V = 0.5 a × *b²* where a and b are the long and short diameters of the tumor, respectively. Tumor growth inhibition (TGI) were calculated using the formula TGI_{day}(%)=[1-(V_{treated -day}-V_{treated-day0})/(V_{control-day}-V_{control-day0})]×100, where V_{treated-day0} and V_{control-day0} are the mean volumes of the treated and control groups at day0 of the study (grouping day); and V_{treated-day} and V_{control-day} are the mean volumes of the treated and control groups on a given day. Mice were sacrificed when the tumor volume exceeded 2000 mm³ or in case of ulceration of the tumor. Control ADC cantuzumab ravtansine was purchased from MedChemExpress (cat#HY-P99493).

**Table 6A: tumor growth inhibition (TGI) of ADCs tested in HT-29 xenograft study**

| **ADC name** | **DAR value** | **ADC dose (mg/kg)** | **Conjugated drug (payload) dose (nmol/kg)** | **TGI (%) at day 24** |
|---|---|---|---|---|
| VH3VL4_LALA_DL1_DAR2 | 2 | 10 | 129 | 85.5 |
| | | 20 | 258 | 113.9 |
| | | 30 | 387 | 121.8 |
| VH3VL4_LALA_DL3_DAR2 | 2 | 10 | 129 | 105.2 |
| | | 20 | 258 | 110.4 |
| | | 30 | 387 | 118.8 |
| VH3VL4_LALA_DL4_DAR2 | 2 | 10 | 129 | 34.5 |
| | | 20 | 258 | 23.5 |
| | | 30 | 387 | 65.6 |
| NEGIGG_LALA_DL1_DAR2 | 2 | 10 | 129 | 8.5 |
| | | 20 | 258 | -97.5 |
| | | 30 | 387 | -78.1 |
| CANTUZUMAB_DM4 | 4 | 5 | 129 | -37.9 |
| | | 10 | 258 | 120.5 |
| | | 15 | 387 | 125.6 |

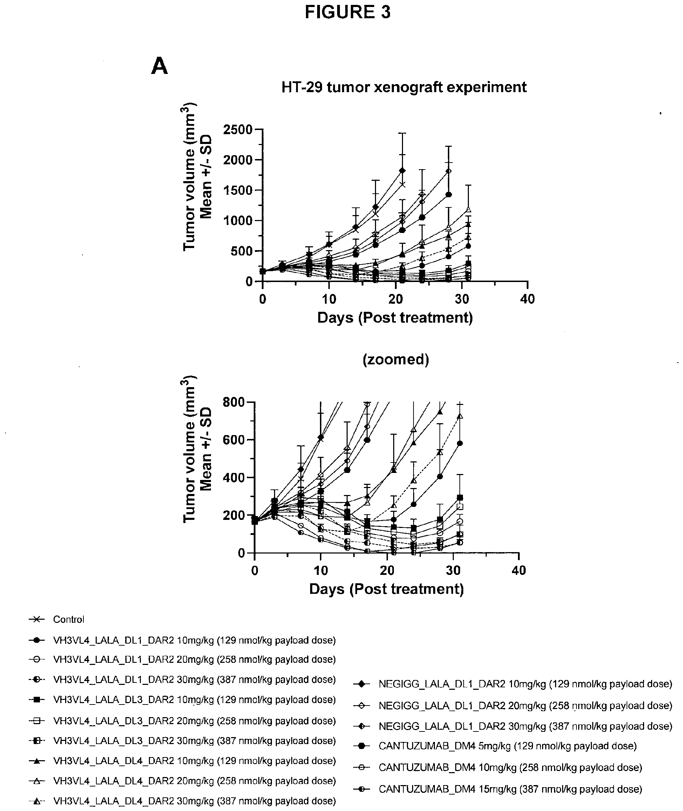

The *in vivo* efficacy of ADCs on HT-29 tumor model is presented in Figure 3A. No significant body weight loss or apparent signs of toxicity was observed during this study.

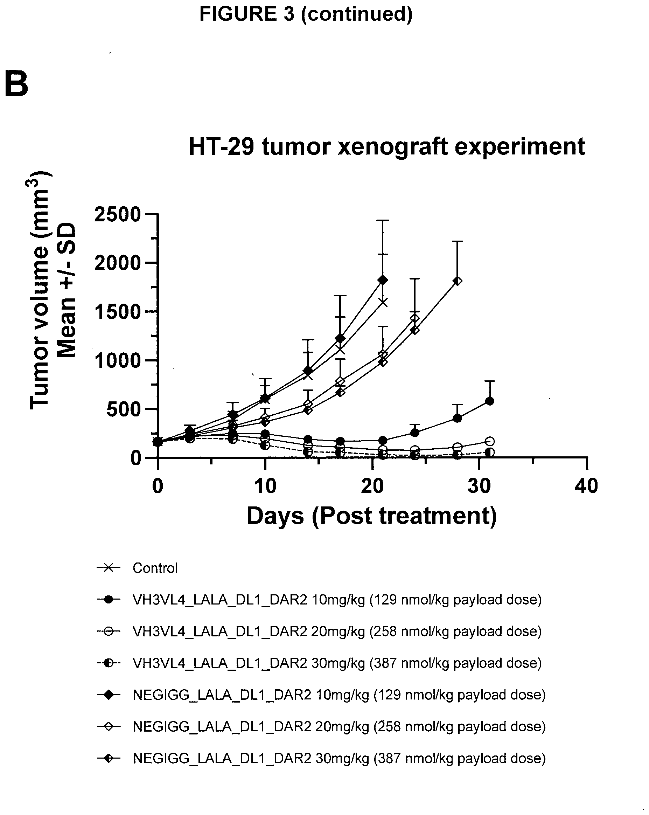

Non-targeted non binding control ADC NEGIGG_LALA_DL1_DAR2 showed limited antitumor efficacy compared to VH3VL4_LALA_DL1_DAR2 at every doses (see Figure 3B). This shows that antitumor efficacy is CA242-target mediated.

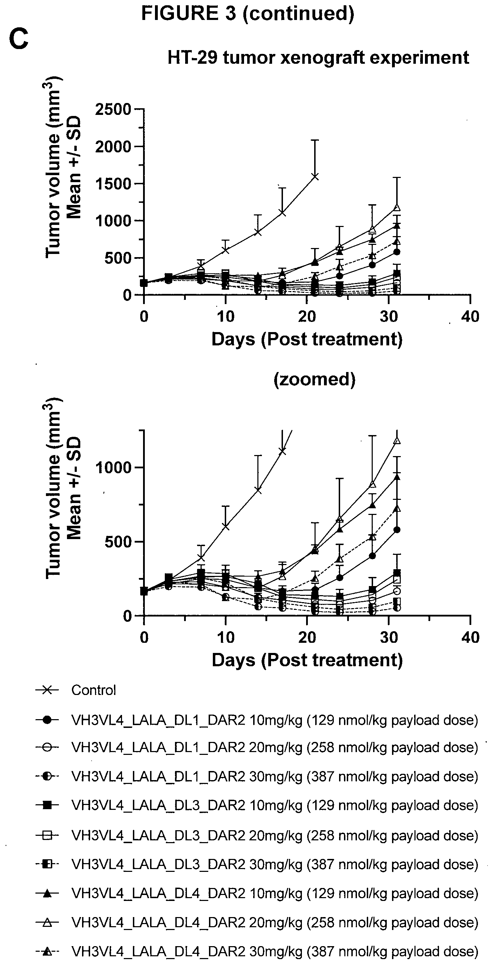

DL1 and DL3-based ADCs exhibited similar efficacy, whereas DL4-based ADCs were comparatively less efficacious (see Figure 3C).

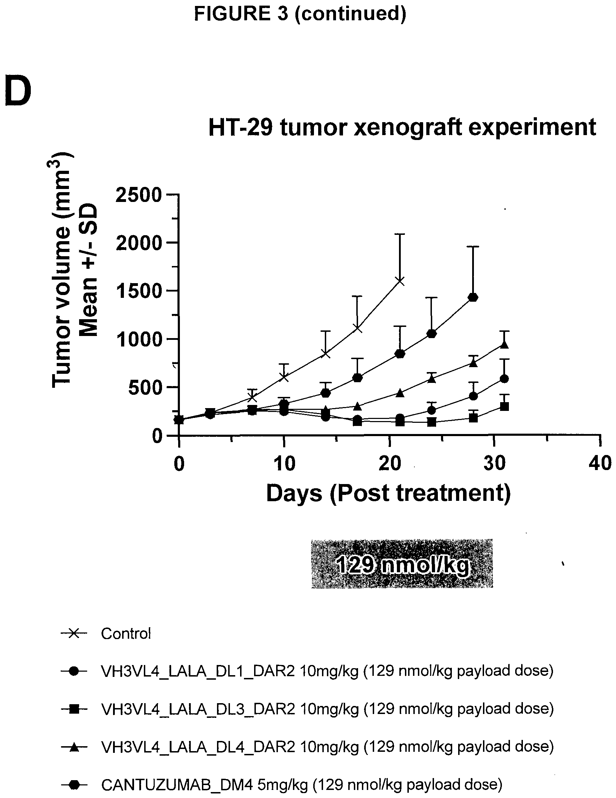

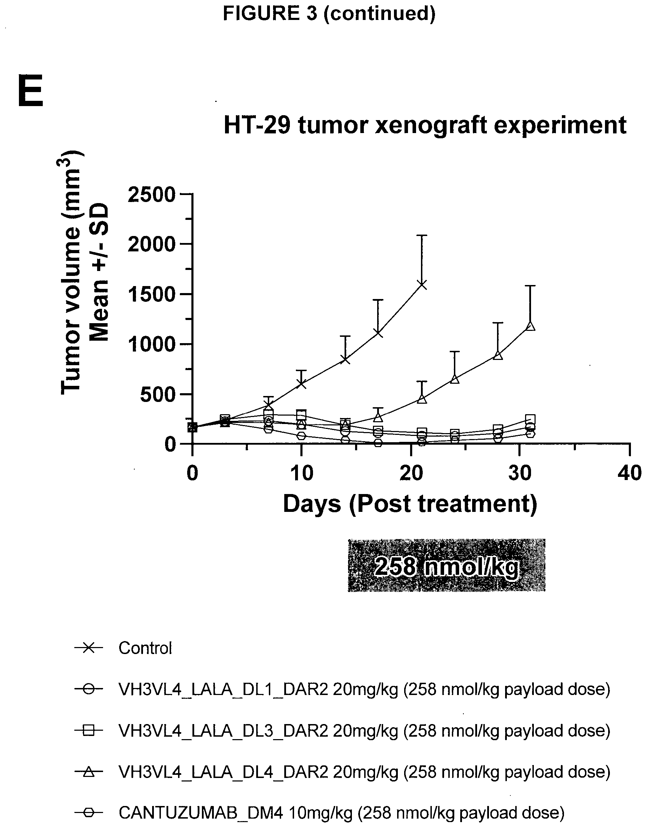

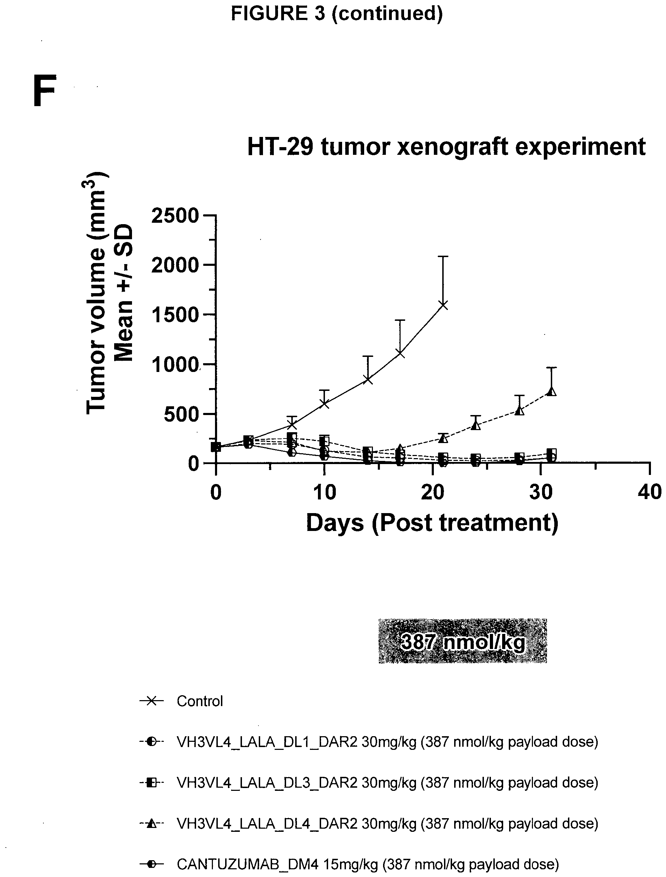

At equivalent 129 nmol/kg conjugated drug dose (see table above, 10mg/kg of DAR2 ADCs according to the present disclosure *versus* 5mg/kg of comparator DAR4 cantuzumab-DM4), ADCs according to the present disclosure are more efficacious (see Figure 3D). At mid and high 258 and 387 nmol/kg conjugated drug dose (20 or 30mg/kg of DAR2 ADCs according to the present disclosure *versus* 10 or 15mg/kg of comparator DAR4 cantuzumab-DM4), DL1 or DL3-based ADCs according to the present disclosure and cantuzumab-DM4 are comparable in terms of efficacy and DL4-based ADCs according to the present disclosure are less efficacious than cantuzumab-DM4 (see Figures 3E and 3F). Table 6B below summarizes those results.

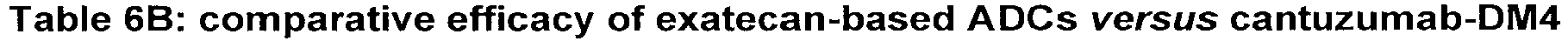

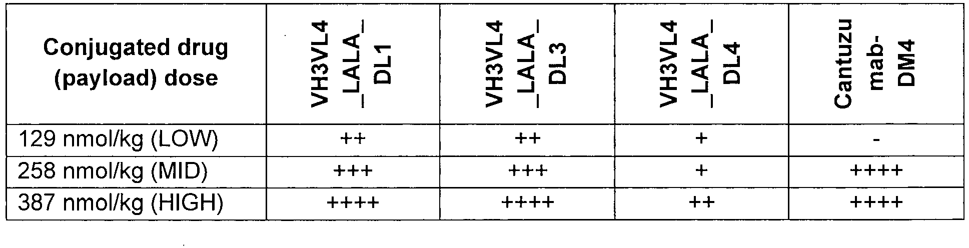

### Sequence Listing

**Table 3: Sequence listings of monoclonal antibodies**

| **SEQ ID** | **Description** | **Sequence** |
|---|---|---|
| SEQ ID NO: 1 | Murine anti-CA242 CDR-H1 | DYTFTYYG |
| SEQ ID NO: 2 | Murine anti-CA242 CDR-H2 | IDTTTGEP |
| SEQ ID NO: 3 | Murine anti-CA242 CDR-H3 | ARRGPYNWYFDV |
| SEQ ID NO: 4 | Murine anti-CA242 CDR-L1 | KSLLHSNGNTY |
| SEQ ID NO: 5 | Murine anti-CA242 CDR-L2 | RMS |
| SEQ ID NO: 6 | Murine anti-CA242 CDR-L3 | LQHLEYPFT |
| SEQ ID NO: 7 | Murine anti-CA242 VH from US5552293A | QVQLVQSGPELKKPGETVKISCKASDYTFTYYG MNWVKQAPGKGLKWMGWIDTTTGEPTYAEDF KGRIAFSLETSASTAYLQIKNLKNEDTATYFCAR RGPYNWYFDVWGAGTTVTVSS |
| SEQ ID NO: 8 | Murine anti-CA242 VL from US5552293A | DIVMTQAAPSVPVTPGESVSISCRSSKSLLHSN GNTYLYWFLQRPGQSPQLLIYRMSNLVSGVPD RFSGSGSGTAFTLRISRVEAEDVGVYYCLQHLE YPFTFGPGTKLELK |
| SEQ ID NO: 9 | Murine anti-CA242 VH from US5552293A in hlgG1_LALA_A118 C HC backbone (chimeric control antibody) | QVQLVQSGPELKKPGETVKISCKASDYTFTYYG MNWVKQAPGKGLKWMGWIDTTTGEPTYAEDF KGRIAFSLETSASTAYLQIKNLKNEDTATYFCAR RGPYNWYFDVWGAGTTVTVSSCSTKGPSVFPL APSSKSTSGGTAALGCLVKDYFPEPVTVSWNS GALTSGVHTFPAVLQSSGLYSLSSVVTVPSSSL GTQTYICNVNHKPSNTKVDKKVEPKSCDKTHTC PPCPAPEAAGGPSVFLFPPKPKDTLMISRTPEV TCVVVDVSHEDPEVKFNWYVDGVEVHNAKTKP REEQYNSTYRVVSVLTVLHQDWLNGKEYKCKV SNKALPAPIEKTISKAKGQPREPQVYTLPPSRDE LTKNQVSLTCLVKGFYPSDIAVEWESNGQPENN |
| | | YKTTPPVLDSDGSFFLYSKLTVDKSRWQQGNV FSCSVMHEALHNHYTQKSLSLSPGK |
| SEQ ID NO: 10 | Murine anti-CA242 VL from US5552293A in hIgG1 LC backbone (chimeric control antibody) | DIVMTQAAPSVPVTPGESVSISCRSSKSLLHSN GNTYLYWFLQRPGQSPQLLIYRMSNLVSGVPD RFSGSGSGTAFTLRISRVEAEDVGVYYCLQHLE YPFTFGPGTKLELKRTVAAPSVFIFPPSDEQLKS GTASVVCLLNNFYPREAKVQWKVDNALQSGNS QESVTEQDSKDSTYSLSSTLTLSKADYEKHKVY ACEVTHQGLSSPVTKSFNRGEC |
| SEQ ID NO: 11 | Murine anti-CA242 VH from US5552293A in mulgG2a backbone | QVQLVQSGPELKKPGETVKISCKASDYTFTYYG MNWVKQAPGKGLKWMGWIDTTTGEPTYAEDF KGRIAFSLETSASTAYLQIKNLKNEDTATYFCAR RGPYNWYFDVWGAGTTVTVSSAKTTAPSVYPL APVCGDTTGSSVTLGCLVKGYFPEPVTLTWNS GSLSSGVHTFPAVLQSDLYTLSSSVTVTSSTWP SQSITCNVAHPASSTKVDKKIEPRGPTIKPCPPC KCPAPNLLGGPSVFIFPPKIKDVLMISLSPIVTCV VVDVSEDDPDVQISWFVNNVEVHTAQTQTHRE DYNSTLRVVSALPIQHQDWMSGKEFKCKVNNK DLPAPIERTISKPKGSVRAPQVYVLPPPEEEMTK KQVTLTCMVTDFMPEDIYVEWTNNGKTELNYK NTEPVLDSDGSYFMYSKLRVEKKNWVERNSYS CSVVHEGLHNHHTTKSFSRTPGK |
| SEQ ID NO: 12 | Murine anti-CA242 VL from US5552293A in mulgG2a backbone | DIVMTQAAPSVPVTPGESVSISCRSSKSLLHSN GNTYLYWFLQRPGQSPQLLIYRMSNLVSGVPD RFSGSGSGTAFTLRISRVEAEDVGVYYCLQHLE YPFTFGPGTKLELKRADAAPTVSIFPPSSEQLTS GGASVVCFLNNFYPKDINVKWKIDGSERQNGVL NSWTDQDSKDSTYSMSSTLTLTKDEYERHNSY TCEATHKTSTSPIVKSFNRNEC |
| SEQ ID NO: 13 | Humanized anti-CA242 VH1 | QVQLQQSGPGLVKPSETLSLTCTASDYTFTYYG MNWVRQPPGKGLEWMGWIDTTTGEPTYAESL KSRITFSLETSASTASLKLSSVTAADTAVYFCAR RGPYNWYFDVWGQGTTVTVSS |
| SEQ ID NO: 14 | Humanized anti-CA242 VH2 | QVQLQESGPGLVKPSETLSLTCTASDYTFTYYG MNWIRQPPGKGLEWIGWIDTTTGEPTYAESLKS |
| | | RVTISLDTSKNQASLKLSSVTAADTAVYYCARR GPYNWYFDVWGQGTTVTVSS |
| SEQ ID NO: 15 | Humanized anti-CA242 VH3 | QVQLVQSGAEVKKPGASVKVSCKASDYTFTYY GMNWVRQAPGQRLEWMGWIDTTTGEPTYAEK FQGRITFTLETSASTAYMELSSLRSEDTAVYFCA RRGPYNWYFDVWGQGTTVTVSS |
| SEQ ID NO: 16 | Humanized anti-CA242 VH4 | QVQLVQSGAEVKKPGASVKVSCKASDYTFTYY GMNWVRQAPGQRLEWMGWIDTTTGEPTYAEK FQGRVTITLDTSASTAYMELSSLRSEDTAVYYC ARRGPYNWYFDVWGQGTTVTVSS |
| SEQ ID NO: 17 | Humanized anti-CA242 VH5 | QVQLVQSGAEVKKPGETVKISCKASDYTFTYYG MNWVKQAPGQGLKWMGWIDTTTGEPTYAQKF QGRIAFSLETSASTAYLQIKSLKSEDTATYFCAR RGPYNWYFDVWGQGTTVTVSS |
| SEQ ID NO: 18 | Humanized anti-CA242 VL1 | DIVMTQSPLSLPVTPGEPASISCRSSKSLLHSNG NTYLYWYLQKPGQSPQLLIYRMSNLVSGVPDR FSGSGSGTAFTLKISRVEAEDVGVYYCLQHLEY PFTFGPGTKLEIK |
| SEQ ID NO: 19 | Humanized anti-CA242 VL2 | DIVMTQSPLSLPVTPGEPASISCRSSKSLLHSNG NTYLYWYLQKPGQSPQLLIYRMSNRVSGVPDR FSGSGSGTDFTLKISRVEAEDVGVYYCLQHLEY PFTFGPGTKLEIK |
| SEQ ID NO: 20 | Humanized anti-CA242 VL3 | DIVMTQSPDSLAVSLGERATINCKSSKSLLHSN GNTYLYWYQQKPGQPPKLLIYRMSNLVSGVPD RFSGSGSGTAFTLTISSLQAEDVAVYYCLQHLE YPFTFGPGTKLEIK |
| SEQ ID NO: 21 | Humanized anti-CA242 VL4 | DIVMTQSPDSLAVSLGERATINCKSSKSLLHSN GNTYLYWYQQKPGQPPKLLIYRMSNRVSGVPD RFSGSGSGTDFTLTISSLQAEDVAVYYCLQHLE YPFTFGPGTKLEIK |
| SEQ ID NO: 22 | I Humanized anti-CA242 VL5 | DIVMTQSPLSVPVTPGEPVSISCRSSKSLLHSN GNTYLYWFLQRPGQSPQLLIYRMSNLVSGVPD RFSGSGSGTAFTLRISRVEAEDVGVYYCLQHLE YPFTFGPGTKLELK |
| SEQ ID NO: 23 | I Humanized anti-CA242 | QVQLQQSGPGLVKPSETLSLTCTASDYTFTYYG MNWVRQPPGKGLEWMGWIDTTTGEPTYAESL |
| | VH1_LALA_A118C HC | KSRITFSLETSASTASLKLSSVTAADTAVYFCAR RGPYNWYFDVWGQGTTVTVSSCSTKGPSVFP LAPSSKSTSGGTAALGCLVKDYFPEPVTVSWN SGALTSGVHTFPAVLQSSGLYSLSSVVTVPSSS LGTQTYICNVNHKPSNTKVDKKVEPKSCDKTHT CPPCPAPEAAGGPSVFLFPPKPKDTLMISRTPE VTCVVVDVSHEDPEVKFNWYVDGVEVHNAKTK PREEQYNSTYRVVSVLTVLHQDWLNGKEYKCK VSNKALPAPIEKTISKAKGQPREPQVYTLPPSRD ELTKNQVSLTCLVKGFYPSDIAVEWESNGQPEN NYKTTPPVLDSDGSFFLYSKLTVDKSRWQQGN VFSCSVMHEALHNHYTQKSLSLSPGK |
| SEQ ID NO: 24 | Humanized anti-CA242 VH2_LALA_A118C HC | QVQLQESGPGLVKPSETLSLTCTASDYTFTYYG MNWIRQPPGKGLEWIGWIDTTTGEPTYAESLKS RVTISLDTSKNQASLKLSSVTAADTAVYYCARR GPYNWYFDVWGQGTTVTVSSCSTKGPSVFPLA PSSKSTSGGTAALGCLVKDYFPEPVTVSWNSG ALTSGVHTFPAVLQSSGLYSLSSVVTVPSSSLG TQTYICNVNHKPSNTKVDKKVEPKSCDKTHTCP PCPAPEAAGGPSVFLFPPKPKDTLMISRTPEVT CVVVDVSHEDPEVKFNWYVDGVEVHNAKTKPR EEQYNSTYRVVSVLTVLHQDWLNGKEYKCKVS NKALPAPIEKTISKAKGQPREPQVYTLPPSRDEL TKNQVSLTCLVKGFYPSDIAVEWESNGQPENN YKTTPPVLDSDGSFFLYSKLTVDKSRWQQGNV FSCSVMHEALHNHYTQKSLSLSPGK |
| SEQ ID NO: 25 | Humanized anti-CA242 VH3_LALA_A118C HC | QVQLVQSGAEVKKPGASVKVSCKASDYTFTYY GMNWVRQAPGQRLEWMGWIDTTTGEPTYAEK FQGRITFTLETSASTAYMELSSLRSEDTAVYFCA RRGPYNWYFDVWGQGTTVTVSSCSTKGPSVF PLAPSSKSTSGGTAALGCLVKDYFPEPVTVSW NSGALTSGVHTFPAVLQSSGLYSLSSVVTVPSS SLGTQTYICNVNHKPSNTKVDKKVEPKSCDKTH TCPPCPAPEAAGGPSVFLFPPKPKDTLMISRTP EVTCVVVDVSHEDPEVKFNWYVDGVEVHNAKT KPREEQYNSTYRVVSVLTVLHQDWLNGKEYKC |
| | | KVSNKALPAPIEKTISKAKGQPREPQVYTLPPSR DELTKNQVSLTCLVKGFYPSDIAVEWESNGQPE NNYKTTPPVLDSDGSFFLYSKLTVDKSRWQQG NVFSCSVMHEALHNHYTQKSLSLSPGK |
| SEQ ID NO: 26 | Humanized anti-CA242 VH3_LALA HC | QVQLVQSGAEVKKPGASVKVSCKASDYTFTYY GMNWVRQAPGQRLEWMGWIDTTTGEPTYAEK FQGRITFTLETSASTAYMELSSLRSEDTAVYFCA RRGPYNWYFDVWGQGTTVTVSSASTKGPSVF PLAPSSKSTSGGTAALGCLVKDYFPEPVTVSW NSGALTSGVHTFPAVLQSSGLYSLSSVVTVPSS SLGTQTYICNVNHKPSNTKVDKKVEPKSCDKTH TCPPCPAPEAAGGPSVFLFPPKPKDTLMISRTP EVTCVVVDVSHEDPEVKFNWYVDGVEVHNAKT KPREEQYNSTYRVVSVLTVLHQDWLNGKEYKC KVSNKALPAPIEKTISKAKGQPREPQVYTLPPSR DELTKNQVSLTCLVKGFYPSDIAVEWESNGQPE NNYKTTPPVLDSDGSFFLYSKLTVDKSRWQQG NVFSCSVMHEALHNHYTQKSLSLSPGK |
| SEQ ID NO: 27 | Humanized anti-CA242 VH4_LALA_A118C HC | QVQLVQSGAEVKKPGASVKVSCKASDYTFTYY GMNWVRQAPGQRLEWMGWIDTTTGEPTYAEK FQGRVTITLDTSASTAYMELSSLRSEDTAVYYC ARRGPYNWYFDVWGQGTTVTVSSCSTKGPSV FPLAPSSKSTSGGTAALGCLVKDYFPEPVTVSW NSGALTSGVHTFPAVLQSSGLYSLSSVVTVPSS SLGTQTYICNVNHKPSNTKVDKKVEPKSCDKTH TCPPCPAPEAAGGPSVFLFPPKPKDTLMISRTP EVTCVVVDVSHEDPEVKFNWYVDGVEVHNAKT KPREEQYNSTYRVVSVLTVLHQDWLNGKEYKC KVSNKALPAPIEKTISKAKGQPREPQVYTLPPSR DELTKNQVSLTCLVKGFYPSDIAVEWESNGQPE NNYKTTPPVLDSDGSFFLYSKLTVDKSRWQQG NVFSCSVMHEALHNHYTQKSLSLSPGK |
| SEQ ID NO: 28 | Humanized anti-CA242 VH4_LALA HC | QVQLVQSGAEVKKPGASVKVSCKASDYTFTYY GMNWVRQAPGQRLEWMGWIDTTTGEPTYAEK FQGRVTITLDTSASTAYMELSSLRSEDTAVYYC ARRGPYNWYFDVWGQGTTVTVSSASTKGPSV |
| | | FPLAPSSKSTSGGTAALGCLVKDYFPEPVTVSW NSGALTSGVHTFPAVLQSSGLYSLSSVVTVPSS SLGTQTYICNVNHKPSNTKVDKKVEPKSCDKTH TCPPCPAPEAAGGPSVFLFPPKPKDTLMISRTP EVTCVVVDVSHEDPEVKFNWYVDGVEVHNAKT KPREEQYNSTYRVVSVLTVLHQDWLNGKEYKC KVSNKALPAPIEKTISKAKGQPREPQVYTLPPSR DELTKNQVSLTCLVKGFYPSDIAVEWESNGQPE NNYKTTPPVLDSDGSFFLYSKLTVDKSRWQQG NVFSCSVMHEALHNHYTQKSLSLSPGK |
| SEQ ID NO: 29 | Cantuzumab VH_LALA_A118C HC | QVQLVQSGAEVKKPGETVKISCKASDYTFTYYG MNWVKQAPGQGLKWMGWIDTTTGEPTYAQKF QGRIAFSLETSASTAYLQIKSLKSEDTATYFCAR RGPYNWYFDVWGQGTTVTVSSCSTKGPSVFP LAPSSKSTSGGTAALGCLVKDYFPEPVTVSWN SGALTSGVHTFPAVLQSSGLYSLSSVVTVPSSS LGTQTYICNVNHKPSNTKVDKKVEPKSCDKTHT CPPCPAPEAAGGPSVFLFPPKPKDTLMISRTPE VTCVVVDVSHEDPEVKFNWYVDGVEVHNAKTK PREEQYNSTYRVVSVLTVLHQDWLNGKEYKCK VSNKALPAPIEKTISKAKGQPREPQVYTLPPSRD ELTKNQVSLTCLVKGFYPSDIAVEWESNGQPEN NYKTTPPVLDSDGSFFLYSKLTVDKSRWQQGN VFSCSVMHEALHNHYTQKSLSLSPGK |
| SEQ ID NO: 30 | Cantuzumab VH_LALA HC | QVQLVQSGAEVKKPGETVKISCKASDYTFTYYG MNWVKQAPGQGLKWMGWIDTTTGEPTYAQKF QGRIAFSLETSASTAYLQIKSLKSEDTATYFCAR RGPYNWYFDVWGQGTTVTVSSASTKGPSVFPL APSSKSTSGGTAALGCLVKDYFPEPVTVSWNS GALTSGVHTFPAVLQSSGLYSLSSVVTVPSSSL GTQTYICNVNHKPSNTKVDKKVEPKSCDKTHTC PPCPAPEAAGGPSVFLFPPKPKDTLMISRTPEV TCVVVDVSHEDPEVKFNWYVDGVEVHNAKTKP REEQYNSTYRVVSVLTVLHQDWLNGKEYKCKV SNKALPAPIEKTISKAKGQPREPQVYTLPPSRDE LTKNQVSLTCLVKGFYPSDIAVEWESNGQPENN |
| | | YKTTPPVLDSDGSFFLYSKLTVDKSRWQQGNV FSCSVMHEALHNHYTQKSLSLSPGK |
| SEQ ID NO: 31 | Humanized anti-CA242 VL1 LC | DIVMTQSPLSLPVTPGEPASISCRSSKSLLHSNG NTYLYWYLQKPGQSPQLLIYRMSNLVSGVPDR FSGSGSGTAFTLKISRVEAEDVGVYYCLQHLEY PFTFGPGTKLEIKRTVAAPSVFIFPPSDEQLKSG TASVVCLLNNFYPREAKVQWKVDNALQSGNSQ ESVTEQDSKDSTYSLSSTLTLSKADYEKHKVYA CEVTHQGLSSPVTKSFNRGEC |
| SEQ ID NO: 32 | Humanized anti-CA242 VL2 LC | DIVMTQSPLSLPVTPGEPASISCRSSKSLLHSNG NTYLYWYLQKPGQSPQLLIYRMSNRVSGVPDR FSGSGSGTDFTLKISRVEAEDVGVYYCLQHLEY PFTFGPGTKLEIKRTVAAPSVFIFPPSDEQLKSG TASVVCLLNNFYPREAKVQWKVDNALQSGNSQ ESVTEQDSKDSTYSLSSTLTLSKADYEKHKVYA CEVTHQGLSSPVTKSFNRGEC |
| SEQ ID NO: 33 | Humanized anti-CA242 VL3 LC | DIVMTQSPDSLAVSLGERATINCKSSKSLLHSN GNTYLYWYQQKPGQPPKLLIYRMSNLVSGVPD RFSGSGSGTAFTLTISSLQAEDVAVYYCLQHLE YPFTFGPGTKLEIKRTVAAPSVFIFPPSDEQLKS GTASVVCLLNNFYPREAKVQWKVDNALQSGNS QESVTEQDSKDSTYSLSSTLTLSKADYEKHKVY ACEVTHQGLSSPVTKSFNRGEC |
| SEQ ID NO: 34 | Humanized anti-CA242 VL4 LC | DIVMTQSPDSLAVSLGERATINCKSSKSLLHSN GNTYLYWYQQKPGQPPKLLIYRMSNRVSGVPD RFSGSGSGTDFTLTISSLQAEDVAVYYCLQHLE YPFTFGPGTKLEIKRTVAAPSVFIFPPSDEQLKS GTASVVCLLNNFYPREAKVQWKVDNALQSGNS QESVTEQDSKDSTYSLSSTLTLSKADYEKHKVY ACEVTHQGLSSPVTKSFNRGEC |
| SEQ ID NO: 35 | Humanized anti-CA242 VL4_V205C LC | DIVMTQSPDSLAVSLGERATINCKSSKSLLHSN GNTYLYWYQQKPGQPPKLLIYRMSNRVSGVPD RFSGSGSGTDFTLTISSLQAEDVAVYYCLQHLE YPFTFGPGTKLEIKRTVAAPSVFIFPPSDEQLKS GTASVVCLLNNFYPREAKVQWKVDNALQSGNS |
| | | QESVTEQDSKDSTYSLSSTLTLSKADYEKHKVY ACEVTHQGLSSPCTKSFNRGEC |
| SEQ ID NO: 36 | Cantuzumab LC | DIVMTQSPLSVPVTPGEPVSISCRSSKSLLHSN GNTYLYWFLQRPGQSPQLLIYRMSNLVSGVPD RFSGSGSGTAFTLRISRVEAEDVGVYYCLQHLE YPFTFGPGTKLELKRTVAAPSVFIFPPSDEQLKS GTASVVCLLNNFYPREAKVQWKVDNALQSGNS QESVTEQDSKDSTYSLSSTLTLSKADYEKHKVY ACEVTHQGLSSPVTKSFNRGEC |
| SEQ ID NO: 37 | Cantuzumab VL_V205C LC | DIVMTQSPLSVPVTPGEPVSISCRSSKSLLHSN GNTYLYWFLQRPGQSPQLLIYRMSNLVSGVPD RFSGSGSGTAFTLRISRVEAEDVGVYYCLQHLE YPFTFGPGTKLELKRTVAAPSVFIFPPSDEQLKS GTASVVCLLNNFYPREAKVQWKVDNALQSGNS QESVTEQDSKDSTYSLSSTLTLSKADYEKHKVY ACEVTHQGLSSPCTKSFNRGEC |
| SEQ ID NO: 38 | Negative control non-binding IgG LC_V205C (WuXi Biologics proprietary VL targeting a viral protein) | <WUXI BIOLOGICS PROPRIETARY VL> RTVAAPSVFIFPPSDEQLKSGTASVVCLLNNFYP REAKVQWKVDNALQSGNSQESVTEQDSKDST YSLSSTLTLSKADYEKHKVYACEVTHQGLSSPC TKSFNRGEC |
| SEQ ID NO: 39 | Negative control non-binding IgG HC_LALA (WuXi Biologics proprietary VH targeting a viral protein) | <WUXI BIOLOGICS PROPRIETARY VL>ASTKGPSVFPLAPSSKSTSGGTAALGCLVK DYFPEPVTVSWNSGALTSGVHTFPAVLQSSGL YSLSSVVTVPSSSLGTQTYICNVNHKPSNTKVD KKVEPKSCDKTHTCPPCPAPEAAGGPSVFLFPP KPKDTLMISRTPEVTCVVVDVSHEDPEVKFNWY VDGVEVHNAKTKPREEQYNSTYRVVSVLTVLH QDWLNGKEYKCKVSNKALPAPIEKTISKAKGQP REPQVYTLPPSRDELTKNQVSLTCLVKGFYPSD IAVEWESNGQPENNYKTTPPVLDSDGSFFLYSK LTVDKSRWQQGNVFSCSVMHEALHNHYTQKSL SLSPGK |
| SEQ ID NO: 40 | Negative control non-binding IgG | <WUXI BIOLOGICS PROPRIETARY VL>CSTKGPSVFPLAPSSKSTSGGTAALGCLVK |
| | HC_LALA_A118C (WuXi Biologics proprietary VH targeting a viral protein) | DYFPEPVTVSWNSGALTSGVHTFPAVLQSSGL YSLSSVVTVPSSSLGTQTYICNVNHKPSNTKVD KKVEPKSCDKTHTCPPCPAPEAAGGPSVFLFPP KPKDTLMISRTPEVTCVVVDVSHEDPEVKFNWY VDGVEVHNAKTKPREEQYNSTYRVVSVLTVLH QDWLNGKEYKCKVSNKALPAPIEKTISKAKGQP REPQVYTLPPSRDELTKNQVSLTCLVKGFYPSD IAVEWESNGQPENNYKTTPPVLDSDGSFFLYSK LTVDKSRWQQGNVFSCSVMHEALHNHYTQKSL SLSPGK |
| SEQ ID NO: 41 | LALA HC constant region | ASTKGPSVFPLAPSSKSTSGGTAALGCLVKDYF PEPVTVSWNSGALTSGVHTFPAVLQSSGLYSLS SVVTVPSSSLGTQTYICNVNHKPSNTKVDKKVE PKSCDKTHTCPPCPAPEAAGGPSVFLFPPKPK DTLMISRTPEVTCVVVDVSHEDPEVKFNWYVD GVEVHNAKTKPREEQYNSTYRVVSVLTVLHQD WLNGKEYKCKVSNKALPAPIEKTISKAKGQPRE PQVYTLPPSRDELTKNQVSLTCLVKGFYPSDIA VEWESNGQPENNYKTTPPVLDSDGSFFLYSKL TVDKSRWQQGNVFSCSVMHEALHNHYTQKSL SLSPGK |
| SEQ ID NO: 42 | LALA_A118C HC constant region | CSTKGPSVFPLAPSSKSTSGGTAALGCLVKDYF PEPVTVSWNSGALTSGVHTFPAVLQSSGLYSLS SVVTVPSSSLGTQTYICNVNHKPSNTKVDKKVE PKSCDKTHTCPPCPAPEAAGGPSVFLFPPKPK DTLMISRTPEVTCVVVDVSHEDPEVKFNWYVD GVEVHNAKTKPREEQYNSTYRVVSVLTVLHQD WLNGKEYKCKVSNKALPAPIEKTISKAKGQPRE PQVYTLPPSRDELTKNQVSLTCLVKGFYPSDIA VEWESNGQPENNYKTTPPVLDSDGSFFLYSKL TVDKSRWQQGNVFSCSVMHEALHNHYTQKSL SLSPGK |
| SEQ ID NO: 43 | LC constant region | |
| SEQ ID NO: 44 | V205C LC constant region | RTVAAPSVFIFPPSDEQLKSGTASVVCLLNNFYP REAKVQWKVDNALQSGNSQESVTEQDSKDST YSLSSTLTLSKADYEKHKVYACEVTHQGLSSPC TKSFNRGEC |

### References

| | **Full reference** | **DOI** |
|---|---|---|
| Baeckström 1991 | Baeckström, et al., Journal of Biological Chemistry, 266(32), 21537-21547, 1991 | 10.1016/S0021-9258(18)54672-X |
| Rubahamya 2024 | Rubahamya et al., Science Advances, 2024, 10 (22), eadk189 | 10.1126/sciadv.adk1894 |
| Helft 2004 | Helft et al., Clinical Cancer Research, 10(13), 4363-4368, 2004 | 10.1158/1078-0432.CCR-04-0088 |
| Mita 2007 | Mita et al., Journal of Clinical Oncology, ASCO Annual Meeting Abstract, 25(18_suppl), 3062-3062, 2007 | 10.1200/jco.2007.25.18_suppl.306 |
| O'Connell 1978 | O'Connell et al., Cancer Treatment Reports, 62(8), 1237-1238, 1978 | PMID: 356981 |
| Mysliwy 2022 | Mysliwy et al., AACR Annual Meeting Abstract, Cancer Research, 82(12_Suppl), 1751,2022 | 10.1158/1538-7445.AM2022-1751 |
| Gao 2013 | Gao et al., BMC Biotechnology, 13(55), 2013 | 10.1186/1472-6750-13-55 |
| Lilley 2020 | Lilley et al., Angewandte Chemie, 2020, 59(1), 388-394 | 10.1002/anie.201909933 |
| Dondelinger 2018 | Dondelinger et al., Front Immunol. 2018; 9: 2278. | 10.3389/fimmu.2018.02278 |

US5552293A
WO2023/166322

## Claims

1. An antibody-drug conjugate of formula (I):
Ab-[L-D]*ᵢ* (I)
wherein
Ab is an anti-CA242 antibody or antigen binding fragment thereof comprising a variable heavy chain polypeptide comprising CDR-H1 of SEQ ID NO:1, CDR-H2 of SEQ ID NO:2, CDR-H3 of SEQ ID NO:3 and a variable light chain polypeptide comprising CDR-L1 of SEQ ID NO:4, CDR-L2 of SEQ ID NO:5 and CDR-L3 of SEQ ID NO:6;
L is a linker unit connecting Ab to D;
D is a topoisomerase I inhibitor drug moiety bound to L;
i is an integer ranging from 1 to 8.

2. The antibody-drug conjugate of claim 1, wherein Ab comprises:
a variable heavy chain polypeptide comprising CDR-H1, CDR-H2, and CDR-H3 of SEQ ID NO:7, and
a variable light chain polypeptide comprising CDR-L1, CDR-L2, and CDR-L3 of SEQ ID NO:8.

3. The antibody-drug conjugate of claim 1, wherein Ab comprises:
a variable heavy chain polypeptide selected from VH of SEQ ID NO:13, SEQ ID NO:14, SEQ ID NO:15, SEQ ID NO:16 or SEQ ID NO:17; and
a variable light chain polypeptide selected from VL of SEQ ID NO:18, SEQ ID NO:19, SEQ ID NO:20, SEQ ID NO:21 or SEQ ID NO:22.

4. The antibody-drug conjugate of claim 1, wherein Ab comprises:
(a) a constant heavy chain polypeptide selected from SEQ ID NO: 41 or SEQ ID NO: 42; and
(b) a constant light chain polypeptide selected from SEQ ID NO: 43 or SEQ ID NO: 44.

5. The antibody-drug conjugate of claim 1, wherein Ab comprises:
(a) a heavy chain polypeptide selected from SEQ ID NO: 23, SEQ ID NO: 24, SEQ ID NO: 25, SEQ ID NO: 26, SEQ ID NO: 27 or SEQ ID NO: 28; and
(b) a light chain polypeptide selected from SEQ ID NO: 31, SEQ ID NO: 32, SEQ ID NO: 33, SEQ ID NO: 34, or SEQ ID NO: 35.

6. The antibody-drug conjugate of claim 5, wherein Ab comprises:
(a) a constant heavy chain polypeptide of SEQ ID NO: 26; and
(b) a constant light chain polypeptide of SEQ ID NO: 35.

7. The antibody-drug conjugate of any one of claims 1 to 6, wherein D is selected from 7-Ethyl-10-hydroxy-camptothecin, exatecan, exatecan mesylate, *N*-glycyl-exatecan, DXd, irinotecan, etirinotecan, topotecan, gimatecan, belotecan, deruxtecan, belotecan, rubitecan, lurtotecan, diflomotecan, karenitecan, silatecan, namitecan, elomotecan, delimotecan, chimmitecan, indotecan, indimitecan, AZ14170132, Ed-04, ZD06519, YL0014, SHR9265, KL610023, FL118, DRF-1042, NSC606985, ZBH-1205, Genz-644282, SHR9265, A1.9, P1003, P1021, VIP126, ZBH-01, indenoisoquinoline and fluoroindenoisoquinoline.

8. The antibody-drug conjugate of claim 7, wherein D is selected from exatecan, belotecan, SN38 and DXd.

9. The antibody-drug conjugate of claim 7, wherein D is of formula (IIa):

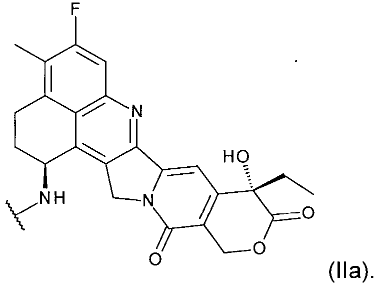

10. The antibody-drug conjugate of any one of claims 1 to 9, wherein L is a linker moiety of formula -W-Y-, wherein W is a stretcher unit linked to Ab, and Y is a cleavable unit linked to D.

11. The antibody-drug conjugate of claim 10, wherein the stretcher unit (W) is selected from:
| | |
|---|---|
| a) | 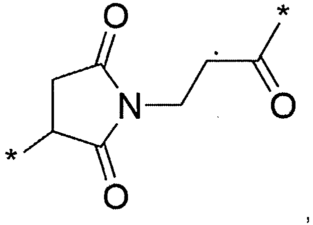 |
| b) | 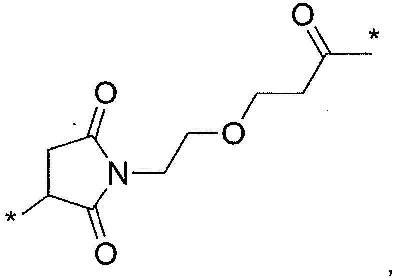 |
| c) | 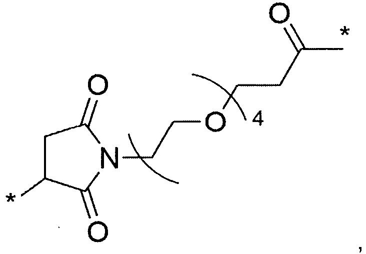 and |
| d) | 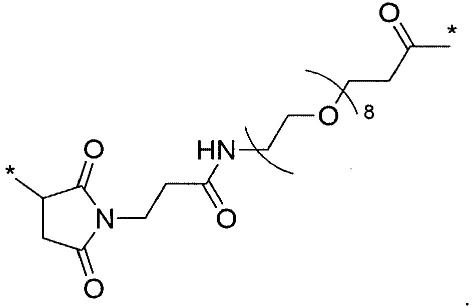 |

12. The antibody-drug conjugate of either claim 10 or claim 11, wherein L is selected from:
| | |
|---|---|
| a) | 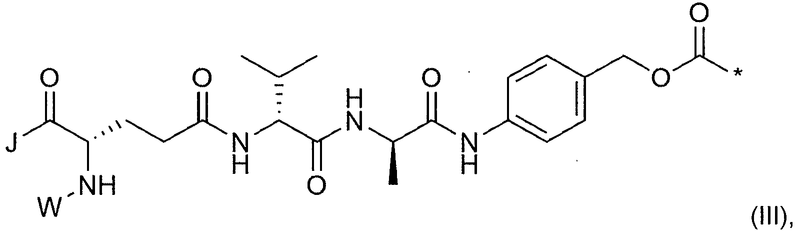 wherein J is OH or -NH-(C₂H₄O)_{f1}-Me, where f1 is from 1 to 6; |
| b) | 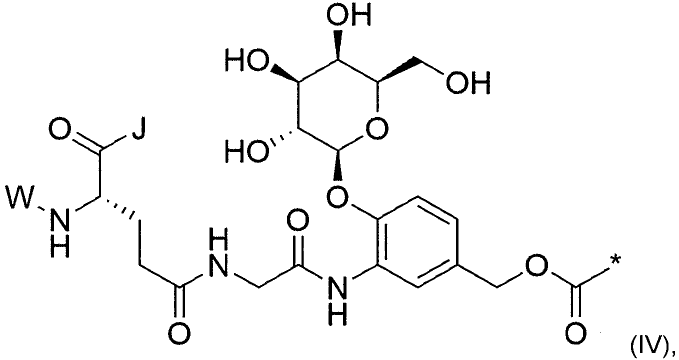 wherein J is OH or -NH-(C₂H₄O)_{f2}-Me, where f2 is from 1 to 6; and |
| c) | 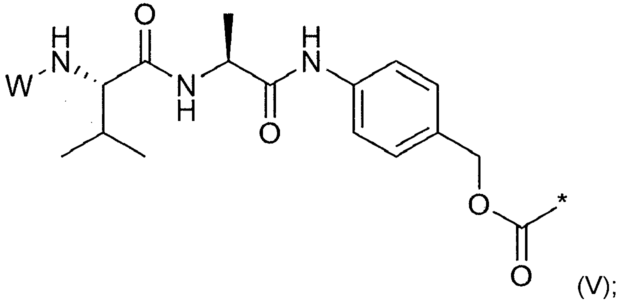 |
where * represents the attachment to the drug and W is as defined above.

13. The antibody drug conjugate according to any one of claims 1 to 6, which is selected from: 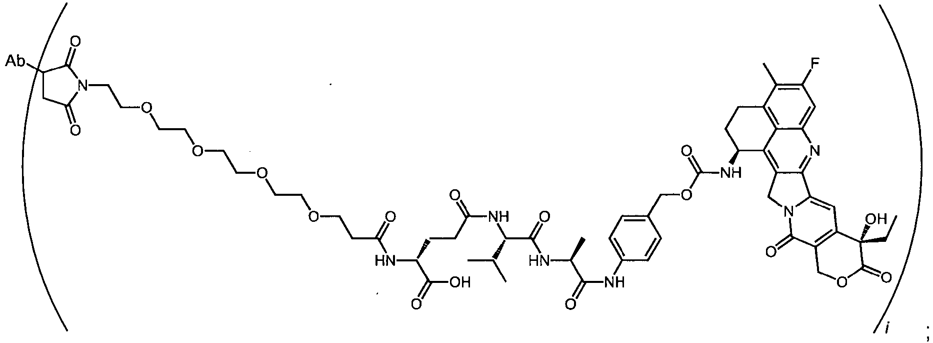 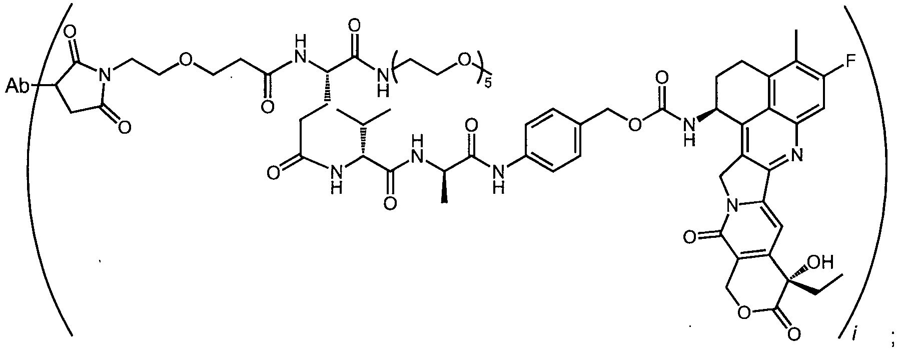 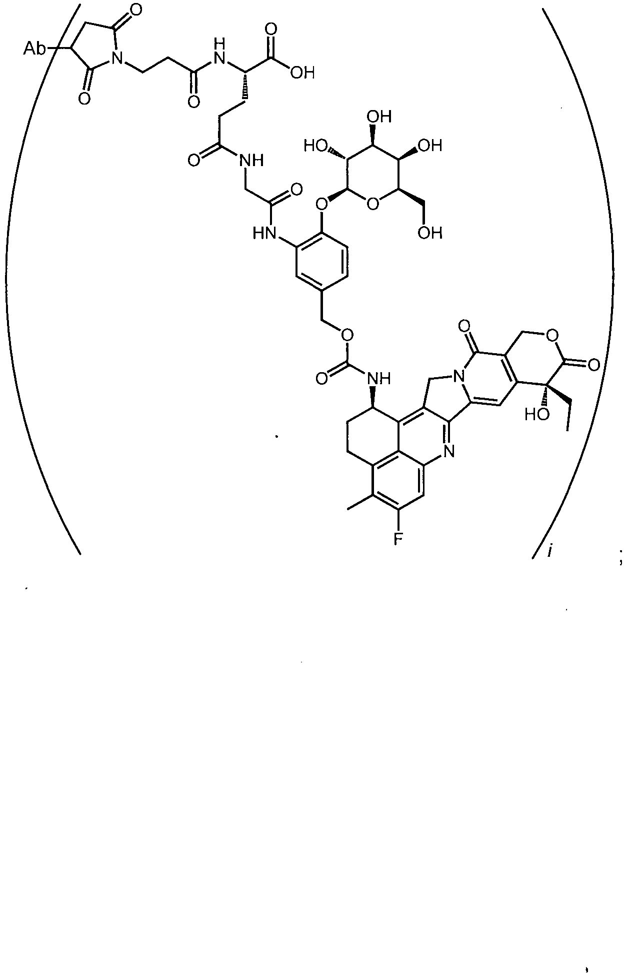 and

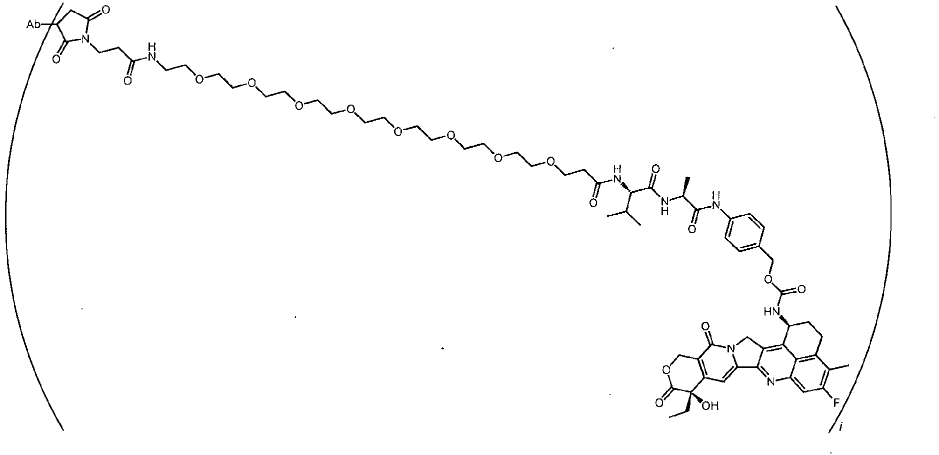

14. The antibody-drug conjugate according to any one of claims 1 to 13, wherein i is an integer from 2 to 4.

15. A pharmaceutical composition comprising an antibody drug conjugate according to any one of claims 1 to 14, and one or more pharmaceutically acceptable excipients, diluents, or carriers.
